(19) **Europäisches Patentamt European Patent Office Office européen des brevets**

(11) **EP 3 265 579 B1**

(12) **FASCICULE DE BREVET EUROPEEN**

(45) Date de publication et mention de la délivrance du brevet:
**12.12.2018 Bulletin 2018/50**

(51) Int Cl.:
*C12Q 1/66* *(2006.01)*   *G01N 33/50* *(2006.01)*

(21) Numéro de dépôt: **16714989.7**

(22) Date de dépôt: **01.03.2016**

(86) Numéro de dépôt international:
**PCT/FR2016/050458**

(87) Numéro de publication internationale:
**WO 2016/139415 (09.09.2016 Gazette 2016/36)**

(54) **PROCÉDÉ NON RADIOACTIF DE DÉTERMINATION DE L'ACTION CYTOLYTIQUE D'UN AGENT VIS-À-VIS DE CELLULES CIBLES, SON UTILISATION ET KIT ASSOCIÉ**

NICHTRADIOAKTIVES VERFAHREN ZUR BESTIMMUNG DER ZYTOLYTISCHEN WIRKUNG EINES MITTELS MIT BEZUG AUF ZIELZELLEN, VERWENDUNG DAVON UND ENTSPRECHENDES KIT

NON-RADIOACTIVE METHOD FOR DETERMINING THE CYTOLYTIC ACTIVITY OF AN AGENT WITH RESPECT TO TARGET CELLS, USE THEREOF AND ASSOCIATED KIT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.03.2015 FR 1551835**
**28.07.2015 FR 1557175**

(43) Date de publication de la demande:
**10.01.2018 Bulletin 2018/02**

(73) Titulaire: **Clean Cells**
**85600 Bouffere (FR)**

(72) Inventeurs:
• **BONNAUDET, Véronique**
  **85600 Saint Hilaire De Loulay (FR)**
• **BRETAUDEAU, Laurent**
  **44690 Maisdon Sur Sevre (FR)**
• **ROSSIGNOL, Alexis**
  **85600 ST GEORGES DE MONTAIGU (FR)**

(74) Mandataire: **Le Cloirec, Claudine et al**
  **Ipsilon**
  **3, rue Edouard Nignon**
  **44300 Nantes (FR)**

(56) Documents cités:
• **XINPING FU ET AL: "A Simple and Sensitive Method for Measuring Tumor-Specific T Cell Cytotoxicity", PLOS ONE, vol. 5, no. 7, 29 juillet 2010 (2010-07-29), page e11867, XP055229329, DOI: 10.1371/journal.pone.0011867**
• **M. D. ALPERT ET AL: "A Novel Assay for Antibody-Dependent Cell-Mediated Cytotoxicity against HIV-1- or SIV-Infected Cells Reveals Incomplete Overlap with Antibodies Measured by Neutralization and Binding Assays", JOURNAL OF VIROLOGY., vol. 86, no. 22, 15 novembre 2012 (2012-11-15), pages 12039-12052, XP055229324, US ISSN: 0022-538X, DOI: 10.1128/JVI.01650-12**
• **INOUYE SATOSHI ET AL: "Unconventional secretion of the mutated 19kDa protein ofOplophorusluciferase (nanoKAZ) in mammalian cells", BIOCHEMICAL AND BIOPHYSICAL RESEARCH COMMUNICATIONS, ACADEMIC PRESS INC. ORLANDO, FL, US, vol. 450, no. 4, 11 juillet 2014 (2014-07-11), pages 1313-1319, XP029044904, ISSN: 0006-291X, DOI: 10.1016/J.BBRC.2014.06.140**
• **J. M. COPPOLA ET AL: "Noninvasive Imaging of Apoptosis and Its Application in Cancer Therapeutics", CLINICAL CANCER RESEARCH, vol. 14, no. 8, 27 mars 2008 (2008-03-27), pages 2492-2501, XP055229359, US ISSN: 1078-0432, DOI: 10.1158/1078-0432.CCR-07-0782**

**Description**

La présente invention concerne le domaine de la mesure de la mort cellulaire et plus particulièrement un procédé de détermination de l'action cytolytique de substances actives vis-à-vis de cellules cibles et/ou d'un milieu environnant lesdites cellules cibles.

Dans le domaine industriel, la réglementation impose de caractériser de la façon la plus complète possible tout phénomène ou produit pouvant avoir des conséquences sur la santé des populations humaines ou animales. Dans le vaste cadre de la mise en place de tests mesurant la mort cellulaire, appelés tests de cytotoxicité, ces phénomènes ou produits peuvent regrouper des phénomènes environnementaux, tels qu'une exposition à une source d'irradiation ou de pollution, des produits chimiques ou biologiques non destinés à la santé ou encore des produits à visée thérapeutique.

C'est dans le domaine des substances biologiquement actives, et plus spécifiquement dans le cadre de substances à destination thérapeutique, que les obligations de caractérisation sont les plus poussées. L'un des principaux critères à caractériser est celui de l'activité biologique, également appelée "potency". En effet, d'après la définition réglementaire de l'agence européenne de médecine (CHMP/BWP/157653/2007), la potency est la mesure quantitative d'une activité biologique basée sur un attribut du produit pharmaceutique lié à une propriété biologique pertinente de celui-ci, cette mesure devant refléter son activité biologique dans la situation clinique pour laquelle le produit est conçu. Selon la réglementation, un test de potency doit donc reposer sur la mesure directe de la fonction biologique attendue du produit testé, cohérente avec son mode d'action connu ou supposé. En d'autres termes, et dans le cas d'un produit dont le but est d'entraîner la mort d'une population cellulaire donnée (la mort de cellules tumorales dans le cas d'un traitement anti-cancéreux, par exemple), un test de cytotoxicité doit reposer sur la mesure directe de la mort de la population cellulaire cible. De la même façon, la mise en place d'un test recherchant l'absence (ou l'existence) d'effets cytotoxiques sur un produit non destiné à un usage thérapeutique doit lui aussi reposer sur une méthode détectant spécifiquement la mort des cellules cibles.

Dans le cas des médicaments biologiques, en particulier celui des anticorps (ou immunoglobulines, notées Ig) à usage thérapeutique, les tests de cytotoxicité figurent parmi la longue liste des tests de contrôle qualité requis par la réglementation avant la mise sur le marché de ces molécules.

D'après les textes réglementaires, ces essais doivent refléter le plus possible l'activité biologique du médicament dans son utilisation clinique, en se basant sur ses mécanismes d'action connus ou supposés. Or les modes d'action des anticorps sont variés :

- Mécanismes dépendant de la région Fab de l'anticorps : action directe des anticorps par leur activité de liaison à l'antigène. Ces mécanismes sont spécifiques à chaque anticorps et peuvent correspondre à i) la neutralisation d'un antigène, ii) la neutralisation d'un antigène membranaire par antagonisme ou iii) l'action agoniste sur un antigène membranaire. Dans ces deux derniers cas, l'action agoniste ou antagoniste sur un antigène membranaire peut conduire à une inhibition de la croissance de la population cellulaire cible et/ou à l'induction de sa mort par apoptose et/ou nécrose.

- Mécanismes effecteurs médiés par la région Fc : mécanismes communs à tous les anticorps, leur intensité dépend de la classe et de la sous-classe des anticorps ainsi que de leur structure physicochimique (séquence en acides aminés, structure et composition des chaînes glycosylées, etc.). Ces fonctions effectrices médiées par la région Fc vont dépendre de l'interaction de ladite région Fc avec deux grands types de récepteurs spécifiques du système immunitaire : i) le système du complément via l'interaction entre la région Fc et le premier composant C1 de la cascade protéolytique du complément ; ii) les récepteurs aux régions Fc des anticorps pour les Ig (notés FcR).

Dans de nombreux cas, en particulier lorsque l'anticorps est dirigé contre une molécule membranaire, le mécanisme recherché pour observer l'effet thérapeutique est de type lytique (par exemple, dans le cas d'une utilisation en onco-hématologie). La lyse peut être obtenue, par exemple, par induction de l'apoptose/nécrose ou de l'inhibition de la prolifération cellulaire via des phénomènes d'agonisme/antagonisme, par activation du système du complément ou par recrutement de cellules cytotoxiques exprimant en surface l'un des FcR (telles que cellules tueuses naturelles [Natural Killer cell ou NK], macrophages, lymphocytes cytotoxiques, cellules polynucléaires, etc.). Dans de tels cas, les textes réglementaires imposent donc la mise en place d'un test de potency mesurant la lyse cellulaire d'une façon biologiquement pertinente et cohérente avec le mode d'action envisagé *in vivo.*

Un premier but de l'invention est donc de proposer une méthode de mesure directe de la mort de cellules (cytotoxicité) par mesure de la quantité libérée dans le milieu d'une enzyme introduite artificiellement dans lesdites cellules.

**Etat de la technique**

Les tests de cytotoxicité, ou cytolyse, sont très largement utilisés en recherche biologique, clinique et pharmaceutique, pour mesurer la mort cellulaire induite par toute substance biologiquement active, par tout mécanisme biologiquement relevant, afin de cribler une banque de molécules candidates, identifier un mécanisme d'action ou caractériser l'activité biologique d'un produit pharmaceutique par exemple (test de potency).

Un test de cytotoxicité réalisé dans le cadre d'une mesure de potency consiste à mesurer la mort de cellules d'intérêt (généralement dénommées cellules cibles) dans des conditions expérimentales cohérentes avec le mécanisme biologique considéré. Dans le cadre des anticorps thérapeutiques, trois mécanismes d'action principaux peuvent être explorés par ce test : 1) une induction d'apoptose par agonisme ou antagonisme d'une molécule membranaire par l'anticorps ; 2) une activation du système du complément (CDC pour "Complement-Dependent Cytotoxicity") par l'anticorps adsorbé à la cellule cible ; 3) un mécanisme de lyse, par ADCC (pour "Antibody-Dependent Cell-mediated Cytotoxicity") ou par phagocytose, médiée par des effecteurs cellulaires recrutés via l'interaction entre les FcR qu'ils expriment à leur surface et la région Fc des anticorps adsorbés aux cellules cibles. Pour mettre en oeuvre chacun de ces tests, des cellules cibles sont incubées avec l'anticorps considéré en présence, respectivement, 1) du milieu de culture seul, 2) du milieu de culture contenant une source de complément ou 3) du milieu de culture contenant des cellules effectrices appropriées.

Il existe à ce jour plusieurs méthodes de mesure directe de la mort cellulaire, c'est-à-dire dont les données mesurées découlent directement et effectivement de la mort des cellules cibles :

a). méthode de libération d'un isotope radioactif ($^{51}$Cr, $^{111}$In, $^{3}$H). Cette méthode consiste à incuber, préalablement à l'expérience de cytolyse en elle-même, les cellules cibles dans une solution de chromate de sodium, qui pénètre dans les cellules pour se fixer aux protéines intracellulaires. Lorsque les cellules meurent, leur contenu intracellulaire est libéré dans le surnageant. Une mesure de la radioactivité du surnageant permet donc une mesure directe de la quantité de cellules cibles mortes, même si d'autres types de cellules (par exemple des cellules cytotoxiques effectrices) ont été mélangées aux cellules cibles au cours de l'essai, car seules les cellules cibles ont été radiomarquées. Le bruit de fond de la méthode (c'est-à-dire l'intensité du signal en absence de mort cellulaire) est minimal en raison d'une libération spontanée du $^{51}$Cr très faible sur le temps moyen que dure un essai de cytotoxicité (3 à 4 heures au minimum). De plus, de par sa nature radioactive, le signal généré est très intense, ce qui conduit à un rapport signal/bruit élevé (de l'ordre de 5 à 12). La méthode est donc très sensible. Mise au point à la fin des années 1960, cette méthode est encore, à ce jour, la méthode de référence, en raison de sa spécificité vis-à-vis de la mort des cellules cibles et de ses performances élevées notamment en termes de sensibilité. Elle présente en outre de bonnes capacités d'analyse à haut débit du fait de la stabilité du signal sur de longues périodes de temps (plusieurs heures à plusieurs jours). En revanche, elle présente l'inconvénient de nécessiter un temps incompressible de marquage avec le $^{51}$Cr (incubation et lavages) d'environ une à deux heures selon les protocoles, allongeant de façon significative la durée de l'expérience. De plus, le marquage ajoute de la variabilité à l'essai, lié à la quantité et à la qualité des réactifs ajoutés, aux différents temps d'incubation et étapes expérimentales. Enfin, les contraintes réglementaires liées à l'utilisation de la radioactivité sont de plus en plus pesantes et coûteuses, en termes d'autorisation administratives, de gestion des sources, d'élimination des déchets, de droit du travail, de suivi médical ou d'exposition des manipulateurs. Par ailleurs ont été décrites plusieurs variantes de cette méthode, mettant en oeuvre d'autres radio-isotopes, par exemple basées sur l'utilisation de tritium ($^{3}$H) ou d'indium ($^{111}$In) mais impliquant les mêmes contraintes de radioprotection et donc soumises aux mêmes limitations.

b). méthode à la calcéine-acétoxyméthyl (calcéine-AM). Ce composé est perméable aux bicouches lipidiques (donc aux membranes des cellules eucaryotes) grâce à son radical acétoxyméthyl et va donc pénétrer dans les cellules. Une fois à l'intérieur, le radical acétoxyméthyl est clivé par des enzymes (estérases) intracellulaires, restaurant alors les propriétés de fluorescence de la calcéine. La calcéine libérée dans le surnageant sert à mesurer la quantité de cellules lysées. Cette méthode présente d'intéressantes capacités d'analyse à haut débit en évitant les problématiques de radioactivité. En revanche, la libération spontanée de calcéine est très élevée (environ 40% du relargage maximum) traduisant une forte perméabilité de la membrane plasmique à la calcéine, ce qui conduit à un bruit de fond élevé de la méthode. Le signal fluorescent émis par la calcéine étant faible, le rapport signal/bruit est peu favorable, aux alentours de 2,5. Cette méthode présente donc une sensibilité et des performances peu élevées et comporte une étape de marquage des cellules cibles avec la calcéine-AM qui augmente la durée expérimentale globale de l'essai d'une à deux heures et amplifie encore sa variabilité.

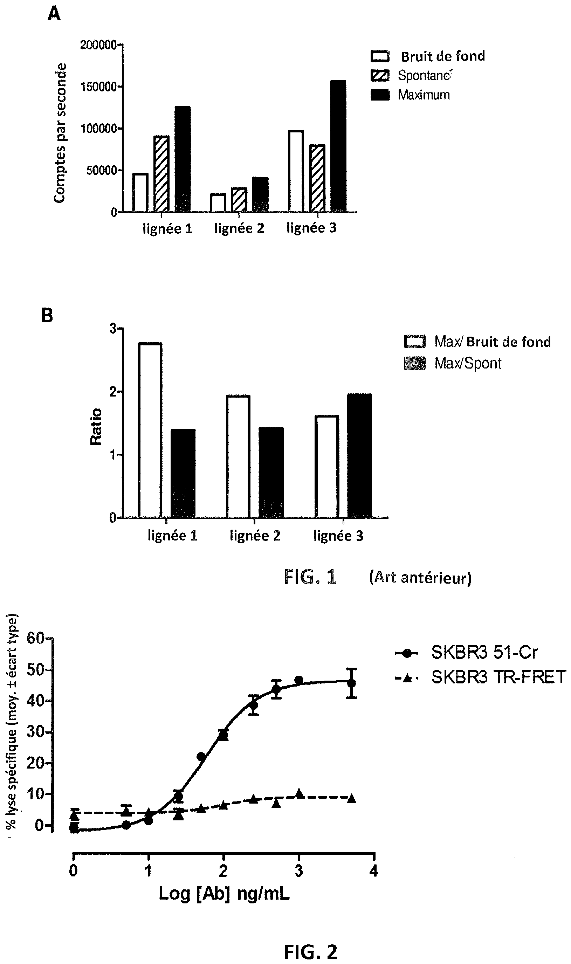

c). méthode basée sur l'utilisation de lanthanides, tels que l'europium (Eu$^{3+}$) ou le terbium (Tb$^{3+}$), complexés à un chélate ou amplificateur de fluorescence tels que le diéthylenetriaminopentaacétate (DTPA) ou l'acide 2,2':6',2"-terpyridine-6,6"-dicarboxylique (TDA), similaires dans leur principe aux méthodes du $^{51}$Cr et de la calcéine-AM. Par exemple, dans la technologie DELFIA (commercialisée par Perkin Elmer, Boston, MA), des cellules cibles sont chargées avec un ligand amplificateur de fluorescence, BATDA (bis [acétoxyméthyl] 2,2':6',2"-terpyridine-6,6"-dicarboxylate) qui pénètre à travers les membranes plasmiques. Dans la cellule, les liaisons esters sont hydrolysées par des estérases pour former un ligand hydrophile, le TDA, en théorie peu perméable à la membrane (après ajout de probenecid, un inhibiteur du MDR, "Multi Drug Resistance" transporteur) et libéré dans le milieu extracellulaire au cours de la cytolyse. Après prélèvement du surnageant, une solution d'europium (Eu) est ajoutée afin que celui-ci se complexe avec le TDA libre pour former un chélate fluorescent (EuTDA). La mesure de ce signal, de type TRF ("Time-Resolved Fluorescence"), est indicatrice de la quantité de cellules lysées. Dans un autre cas, les cellules cibles sont marquées avec l'europium et sa libération dans le surnageant est mesurée par l'ajout du chélate DTPA. Ces méthodes sont non radioactives et présentent une capacité d'analyse à haut débit. En revanche, la libération spontanée de TDA ou d'europium libres est assez élevée et variable selon le type cellulaire : le relargage maximum pour l'$Eu^{3+}$ est le double de celui avec le $^{51}Cr$. Dans certains cas (Figure 1), l'intensité de la libération spontanée est quasi égale à celle du signal spécifique et présente une variabilité dans l'intensité du signal liée au type cellulaire considéré ou à l'état physiologique de la cellule (qui conditionne par exemple la nature et l'activité de ses estérases). Cette méthode nécessite elle aussi un temps expérimental de marquage des cellules cibles avec l'europium ou le BATDA, ainsi qu'un temps d'incubation des surnageants avec le second composant (respectivement TDPA ou $Eu^{3+}$) augmentant la durée expérimentale globale de l'essai et sa variabilité par ajout d'étapes supplémentaires. L'ensemble de ces limitations génère une variabilité de l'essai à l'$Eu^{3+}$ assez élevée, ainsi qu'une impossibilité de l'employer avec un certain nombre de types cellulaires, qui rendent son utilisation et sa validation difficile dans un contexte industriel.

d). méthodes faisant appel à la technique de la cytométrie en flux (et à son dérivé de l'imagerie par cytométrie en flux) pour mesurer la fréquence de cellules vivantes et/ou mortes au cours de l'essai. Ces méthodes ont en commun d'utiliser une combinaison d'un ou plusieurs marqueurs fluorescents pour distinguer les populations de cellules cibles et effectrices. Alternativement, certains auteurs ont utilisé un marquage définitif des cellules cibles en les transformant génétiquement pour exprimer stablement une protéine fluorescente. Ce marquage des populations cibles est couplé à une détection de la viabilité des cellules par l'usage de marqueurs appropriés (généralement des agents intercalants de l'ADN imperméables aux membranes des cellules vivantes mais qui pénètrent dans les cellules apoptotiques ou mortes). La combinaison de ces marqueurs permet de déterminer une fréquence et/ou un nombre de cellules mortes ou vivantes au sein d'une population donnée (généralement les cellules cibles). Ces méthodes, bien que spécifiques du mécanisme de mort cellulaire, ne permettent pas une analyse à haut débit en raison des contraintes techniques liées à la cytométrie en flux. Par exemple, certaines sondes peuvent induire des marquages croisés au cours de la réaction (par échange entre les différents types cellulaires de sondes liées par des liaisons non covalentes). En fonction des méthodes et des équipements, le temps d'acquisition d'un échantillon en cytométrie en flux peut atteindre plusieurs secondes à dizaines de secondes. L'acquisition non simultanée des échantillons qui en résulte a pour conséquence qu'il peut s'écouler plusieurs dizaines de minutes entre le premier et le dernier échantillon d'une série, temps pendant lequel les conditions physiologiques des cellules peuvent évoluer. Ces différents éléments ont des conséquences importantes sur la variabilité des essais et, pour atteindre avec ces méthodes des performances compatibles avec les standards de l'industrie pharmaceutique, l'analyse ne peut dépasser quelques échantillons (en pratique pas plus de trois) testés simultanément, ce qui correspond à un débit faible.

e). méthode basée sur un comptage au microscope par un opérateur ou par un système automatisé. La distinction entre cellules mortes et vivantes et leur dénombrement sont réalisés grâce à l'usage d'un colorant vital, par exemple le bleu trypan ou l'éosine. Néanmoins, ces méthodes microscopiques ne permettent pas de discriminer entre plusieurs types cellulaires distincts qui auraient été mélangés pour les besoins de l'essai (dans le cas d'une mesure d'ADCC par exemple), sauf à utiliser des méthodes de microscopie en fluorescence. On revient alors à des méthodes proches de celles décrites pour la cytométrie en flux mais qui mettent en oeuvre des méthodes de comptage laborieuses et dont les inconvénients déjà cités sont encore amplifiés. Ces méthodes, lentes, peu reproductibles et pas du tout adaptées à une analyse à haut débit, ne sont en pratique pas employées dans le cadre d'essais de potency.

f). variation d'impédance : un certain nombre de méthodes électroniques existent pour mesurer le détachement de cellules adhérentes à un support (ce phénomène pouvant être du à la mort des cellules, de manière non exhaustive). Ces méthodes sont généralement basées sur la mesure d'une variation d'impédance induite par la quantité et/ou l'état physiologique des cellules présentes sur un support adapté. Néanmoins, de telles méthodes ne sont pas adaptées lorsque plusieurs types cellulaires différents sont mélangés et que la mort d'un seul type doit être mesurée (cas des essais d'ADCC par exemple). De plus, ces méthodes nécessitent l'utilisation de cellules cibles adhérentes, un appareillage coûteux, souvent associé à un dispositif expérimental complexe. Et surtout, la mesure du détachement cellulaire ne correspond pas aux mécanismes de cytolyse ; elle peut, au mieux, y être corrélée. Ainsi, leur mise en oeuvre dans un cadre industriel est donc difficile.

g). méthodes basées sur la détection de composants exprimés naturellement dans le cytoplasme des cellules eucaryotes. De telles molécules doivent être libres dans le cytoplasme (c'est-à-dire non incluses dans des vésicules ni liées à des organelles) pour pouvoir être libérées dans le surnageant en cas de lyse de la cellule, mais elles ne doivent pas être sécrétées dans le milieu extracellulaire lorsque les cellules sont dans leur état physiologique. Leur présence dans le surnageant doit être facilement mesurable par des méthodes courantes et elles doivent être fortement exprimées (i.e. un grand nombre de molécules par cellule) et ceci par le plus de types cellulaires différents possibles (molécules ubiquitaires). Il existe un très grand nombre de solutions décrites ou de kits disponibles dans le commerce revendiquant une mesure de la cytotoxicité par de telles méthodes. Les technologies de révélation s'appuient en général sur l'ajout au surnageant d'un ou plusieurs tampons, substrats, enzymes et/ou réactifs permettant de réaliser le dosage en générant une molécule finale mesurable par bioluminescence, chimioluminescence, colorimétrie ou fluorescence. Il est à noter que, dans la plupart des cas, ces tests peuvent également être utilisés "à rebours" pour déterminer la viabilité globale de la population cellulaire. Ces méthodes présentent l'avantage de ne pas nécessiter de marquage particulier des cellules cibles (diminuant le risque de variabilité ainsi que le temps de manipulation) et permettent, de par leur nature, des analyses à débit élevé. Néanmoins, l'inconvénient majeur et rédhibitoire de tous ces tests basés sur la libération d'une molécule ubiquitaire pour mesurer la lyse cellulaire est qu'ils ne sont plus pertinents dès lors que plusieurs types cellulaires sont mélangés pour la réalisation de l'essai. En effet, il est alors impossible de discriminer lequel des types cellulaires a participé, et dans quelle proportion, au relargage de la molécule. C'est par exemple le cas des essais d'ADCC dans lesquels des cellules cibles et des cellules effectrices doivent être mélangées pour observer l'effet cytotoxique. Les cellules effectrices mortes durant l'essai, en lien avec leur taux de mortalité habituel ou par des mécanismes liés à l'essai en lui-même (tels que l'exhaustion ou la lyse redirigée par exemple), vont, elles aussi, participer au signal. En ce sens, il ne s'agit donc pas de méthodes spécifiques à la lyse des cellules cibles. En outre, certaines molécules à mesurer ne sont pas suffisamment stables pendant la durée de l'essai (par exemple l'ATP a une demi-vie très courte dans le milieu extracellulaire), d'autres présentent un bruit de fond élevé et/ou une intensité de signal faible conduisant à un manque de sensibilité de la méthode.

Au vu de ces éléments, un autre but de l'invention est donc de proposer une méthode de cytotoxicité non radioactive, afin de s'affranchir des nombreuses contraintes réglementaires et de santé. NOUYE SATOSHI ET AL: "Unconventional sécrétion of the mutated 19kDa protein of Oplophorus luciferase (nanoKAZ) in mammalian cells",BIOCHEMICAL AND BIOPHYSICAL RESEARCH COMMUNICATIONS, ACADEMIC PRESS INC. ORLANDO, FL, US, vol. 450, no. 4, 11 juillet 2014 (2014-07-11), pages 1313-1319, divulgue l'enzyme Nanokaz, qui est un luciférase de l'organisme Oplophorus; mais celui-ci n'a pas été utilisé dans le contexte d'un essai cytolytique.
J. M. Coppola ET AL: "Noninvasive Imaging of Apoptosis and Its Application in Cancer Therapeutics",CLINICAL CANCER RESEARCH, vol. 14, no. 8, 27 mars 2008 (2008-03-27), pages 2492-2501, divulgue l'utilisation d'un procédé tel que mentionné sous le point 3.1 (avec la différence qu'un peptide avec activité de luciférase ANLuc-DEVD-BCLuc est utilisé au lieu d'un luciférase intégral) pour mesurer la cytotoxicité cellulaire dépendante d'anticorps (ADCC), mesurer la cytotoxicité dépendante du complément (CDC) et/ou mesurer l'apoptose.
Un autre but de l'invention de proposer une méthode de cytotoxicité ne nécessitant pas un marquage extemporané de la cellule cible (afin de ne pas impliquer une perte de temps expérimental et une augmentation de la variabilité globale de l'essai par ajout de plusieurs étapes variables), tout en conservant une mesure directe et spécifique de la lyse de ladite cellule.
Pour pallier les inconvénients précités et contourner la nécessité d'un marquage extemporané de la cellule cible, tout en conservant une mesure directe et spécifique de la lyse de ladite cellule, Schäfer et al. ont décrit en 1997 (Journal of Immunological Methods, 204 pp 89-98, 1997) une méthode de transformation génétique pour obtenir des lignées de cellules cibles exprimant stablement une enzyme exogène intra-cytoplasmique. Dans cette méthode, la mort desdites cellules devait en théorie permettre la libération de cette enzyme dans le milieu extracellulaire, la quantité d'enzyme étant alors mesurée par une méthode appropriée. Ces auteurs ont tout d'abord réalisé la transformation d'une lignée cible par le gène de la F-Luc (Firely Luciferase, luciférase de luciole) sous le contrôle du promoteur de la beta-actine. Cependant la durée de vie de la F-Luc dans le milieu extracellulaire est très courte (demi-vie de 30 minutes) et incompatible avec un essai d'ADCC ou de CDC dont le mécanisme d'action nécessite 2 à 4 heures pour atteindre le maximum de cellules lysées. Puis ils ont évalué la transformation des cellules cibles par la beta-galactosidase et comparé ces performances à celle de la méthode au marquage par le chrome radioactif traditionnel. Bien que la beta-galactosidase se révèle plus stable que la F-Luc, et que le rapport signal/bruit soit meilleur avec la beta-galactosidase qu'avec la méthode au chrome radioactif, la méthode avec la beta-galactosidase sous-estime d'environ 30 à 40% la quantité de cellules mortes, probablement à cause d'une libération incomplète de cette enzyme lors de la mort des cellules. Elle n'est donc pas applicable pour mesurer efficacement la mort de cellules cibles dans un test non radioactif de caractérisation d'anticorps thérapeutiques visant à satisfaire aux exigences règlementaires, par exemple dans le cadre de la caractérisation des activités ADCC et CDC.

Plus récemment, d'autres auteurs ont décrit l'utilisation de cellules cibles exprimant constitutivement la F-Luc dans un test d'ADCC, basé sur un principe différent (Alpert et al., J. Virol. 86:12039, 2012 ; Fu et al., PLoS ONE 5:e11867, 2010) : la quantification du signal luminescent servant à évaluer la quantité de cellules vivantes. Il ne s'agit donc pas d'une méthode de mesure directe de la mort des cellules cibles, qui est de plus sujette à certaines limitations. Ainsi, la demi-vie de la F-Luc étant courte, et l'essai étant relativement long (8 heures), l'activité F-Luc est sensible à des variations telles qu'une éventuelle perturbation de l'état physiologique des cellules au cours de l'essai (diminution de la synthèse, augmentation du catabolisme), sans que cette variation soit directement liée à une modification de la mortalité cellulaire et générant de fait un premier niveau de variabilité de la méthode. De même, la mesure se faisant de façon homogène sur la suspension cellulaire lysée après l'essai, il est fort probable que la F-Luc produite au cours de l'essai (ou tout au moins au cours des dernières heures, étant donnée sa courte demi-vie) et libérée par les cellules mortes dans le surnageant va générer un deuxième niveau de variabilité en ajoutant son signal à celui de la F-Luc purement intracellulaire. La présence d'une activité F-Luc résiduelle dans le surnageant a d'ailleurs été relevée par les auteurs. Enfin, cette méthode nécessite des temps expérimentaux très longs (au moins 8 heures) pour que la perte de signal soit suffisamment significative. Cette méthode n'est donc pas applicable à la caractérisation fonctionnelle d'anticorps thérapeutiques dans le cadre d'essais de "potency" conformes aux exigences réglementaires.

Un autre but de l'invention est donc de pallier les inconvénients précités et de proposer une méthode incluant une étape de transformation génétique pour obtenir des lignées de cellules cibles exprimant, de manière stable pendant toute la durée de l'essai, une enzyme exogène intra-cytoplasmique, la mort desdites cellules permettant la libération (quasi-totale) de cette enzyme dans le milieu extracellulaire.

Encore un but de l'invention est de proposer une méthode de mesure directe, sensible et spécifique de la mort des cellules cibles (et non une mesure de la disparition des cellules vivantes), qui soit notamment applicable à la caractérisation fonctionnelle d'anticorps thérapeutiques dans le cadre d'essais de "potency".

Ces buts, ainsi que d'autres sont atteints par le procédé selon la présente invention, non radioactif de détermination (contrôle et quantification) in vitro directe de l'action cytolytique d'un agent actif vis-à-vis de cellules cibles et/ou d'un milieu environnant des cellules cibles, comprenant les étapes successives ci-après :

i)Transformation génétique de cellules cibles pour exprimer une enzyme exogène aux dites cellules cibles,
ii) Exposition des dites cellules cibles génétiquement transformées à l'agent actif et/ou ou au dit milieu environnant à tester, pouvant conduire à la lyse d'au moins une partie des cellules cibles en libérant ladite enzyme exogène dans le milieu extracellulaire,
iii) Mesure de l'activité de l'enzyme exogène libérée lors de la lyse desdites cellules cibles

caractérisé en ce que ladite enzyme exogène est une enzyme de masse molaire inférieure ou égale à 45 kDa, et dont l'activité est détectable par luminescence ou fluorescence, telle que décrite dans les revendications.

Ainsi, la libération de cette enzyme exogène, notamment en raison de sa taille réduite, est représentative de la lyse des cellules cibles et donne des résultats, en termes de bruit de fond, de lyse maximale, de lyse spécifique et de performances globales, similaires ou supérieures à la méthode de référence du $^{51}$Cr, comme cela sera démontré plus loin dans les exemples. Avantageusement, la masse molaire de l'enzyme exogène est inférieure ou égale à 30 kDa, de préférence inférieure ou égale à 25 kDa, de préférence encore inférieure ou égale à 20 kDa.
De manière avantageuse, ladite enzyme exogène présente une activité enzymatique stable dans le milieu extracellulaire des étapes ii) et iii) dudit procédé pendant au moins 3 heures, de préférence pendant au moins 24 heures et de préférence encore pendant au moins 48 heures, à des températures comprises entre 34°C et 40°C.

Ladite enzyme exogène est une luciférase. De plus, ladite enzyme exogène possède une séquence peptidique qui peut présenter au moins 60 % d'homologie, de préférence au moins 80 % d'homologie avec la séquence peptidique sauvage de la sous-unité 19 kDa de la luciférase produite par la crevette *Oplophorus gracilirostris.*

De manière préférée, ladite enzyme exogène est sous une forme à activité et stabilité optimisée, commercialisée par la société Promega sous le nom NanoLuc®, dénommée dans l'ensemble du texte nanoluciférase.

Une telle enzyme est en particulier stable dans le milieu extracellulaire sur des périodes de temps compatibles avec les types d'essai envisagés, c'est-à-dire au moins 24 heures.

La quantité d'enzyme exogène libérée est mesurée par clivage d'un substrat spécifique de ladite enzyme, le substrat étant de préférence de la famille de la coelentérazine, plus particulièrement de la famille de la furimazine et de ses dérivés.

Le milieu à tester environnant les cellules cibles peut comprendre un agent biologique et/ou un agent chimique et/ou un agent physique potentiellement actif(s) vis-à-vis des dites cellules cibles.

Le procédé comporte donc comme première étape i), la transformation génétique d'une lignée pertinente de cellules cibles pour lui faire exprimer stablement ladite enzyme exogène, ségréguée dans le cytoplasme en conditions physiologiques.

Selon un premier mode de réalisation de l'invention, l'agent biologique est un anticorps, de préférence choisi parmi les anticorps monoclonaux, notamment à visée thérapeutique.

Dans le cas des anticorps ou molécules apparentées, le choix de la cellule cible support se fait en fonction de la cible de l'anticorps à tester. A chaque nouveau modèle expérimental (pour une cible antigénique donnée), une nouvelle cellule cible est développée, sauf si une même cible exprime naturellement ou artificiellement plusieurs antigènes pertinents.

Plusieurs options sont possibles pour le choix de ces cellules cibles.

La première option est de sélectionner, parmi les lignées cellulaires disponibles auprès des différentes banques de dépôts de matériel biologique, une lignée pertinente pour le modèle à développer. Quelques unes de ces lignées vont être données à titre d'exemple dans l'alinéa suivant, limitées à quelques cibles antigéniques pour lesquelles il existe actuellement des anticorps commercialisés. Il est bien entendu que d'autres lignées sont possibles, pour les molécules antigéniques citées mais également pour d'autres cibles antigéniques non citées mais pour lesquels des anticorps sont à développer ou en cours de développement.

Dans le cas des anticorps dirigés contre des molécules exprimées principalement par les lymphocytes B normaux ou pathologiques, telles que CD19, CD20 ou IL-6R (CD126), toutes les lignées de type lymphocytes ou lymphomes B sont des candidates possibles, par exemple les lignées WIL2, WIL2-S, Daudi, Raji, Ramos, JY, MC116, GA-10, DOHH, ARH-77, SU-DHL, Z138 ou leurs dérivés. Dans le cas d'anticorps dirigés contre des molécules exprimées principalement par les lymphocytes T normaux ou pathologiques, telles que CD3, CD25 ou LPAM ("Lymphocyte Peyer Patch Adhesion Molecule" ou intégrine alpha[4]beta[7]), toutes les lignées de type lymphocytes ou lymphomes T sont des candidates potentielles, comme par exemple les lignées Jurkat, DERL, HD-MAR-2, HH, SR-786, SUP-T1, Loucy, CCRF-CEM, HUT-78 ou leurs dérivés. Les anticorps peuvent être aussi des anticorps dirigés contre des molécules exprimées principalement par des cellules d'origine lymphoïde normales ou pathologiques, telles que CTLA-4 (CD152), PD-1 (CD279) ou CD30 humaines ; dans ce cas toutes les lignées de lymphocytes B et T seraient des candidats potentielles, donc par exemple toutes les lignées B et T déjà citées précédemment. Les anticorps peuvent être aussi des anticorps dirigés contre des molécules exprimées principalement par des cellules d'origine lymphoïde et myéloïde normales ou pathologiques, telles que CD52, VLA4 (CD49d) ou LFA-1 (CD11a) humaines ; dans ce cas toute lignée cellulaire de lignage lymphoïde ou myéloïde serait appropriée, soit, outre les lignées de lymphocytes B et T déjà citées précédemment, des lignées myéloïdes telles que THP-1, HL-60 ou U-937 ou leurs dérivés, par exemple. Les anticorps peuvent aussi être des anticorps dirigés contre des molécules exprimées principalement par des cellules carcinoïdes, telles que anti-EGFR (pour "Epidermal Growth Factor Receptor", aussi noté HER1 ou ErbB1), EGFR2 (ou HER2, ou ErbB2), EGFR-3 (ou HER3, ou ErbB3) ou EpCAM (CD326) : toute lignée issue de tumeurs carcinoïdes est alors une candidate potentielle, par exemple, HCC1954, SKBR3, SKOV3, Caco-2, HeLa, MCF-7, PC-3 ou leurs dérivés. L'anticorps peut aussi être un anticorps dirigé contre la molécule TNF-alpha : les lignées de lymphocytes T ou de type myéloïde telles que décrites précédemment sont alors des candidates potentielles. L'anticorps peut aussi être un anticorps dirigé contre la molécule VEGF ("Vascular Endothelial Growth Factor") ou son récepteur VEGFR : des lignées décrites pour exprimer ces molécules seraient alors appropriées, par exemple les lignées A375, M21, Hoc-7, Panc-3, D283Med, DAOY, D341Med ou leurs dérivés.

L'autre option possible pour la construction de lignées de cellules cibles pertinentes est de disposer d'une même lignée de cellules exprimant l'enzyme exogène choisie, dans laquelle une ou plusieurs cibles antigénique d'intérêt viendraient être intégrées, dans un second temps, par les techniques classiques de transformation génétique. Dans ce cas, n'importe quelle lignée cellulaire peut convenir pourvu qu'elle ait un potentiel non nul de transformation génétique. De préférence, la lignée choisie n'exprimera pas de potentiels antigènes cibles humains qui pourraient venir perturber l'expression des molécules introduites par génie génétique, et donc augmenter la variabilité d'expression des molécules, et donc augmenter la variabilité générale de l'essai. Par exemple, ces cellules peuvent être d'origine non humaine, telles que les cellules CHO, Sp2/0, NS0, NIH3T3 ou leurs dérivés, ou d'origine humaine embryonnaire telles que les cellules HEK293, IMR-90, NTera2 ou leurs dérivés.

Selon un second mode de réalisation de l'invention, l'agent chimique et/ou physique est choisi parmi les agents de chimiothérapie ou les molécules anti-cancéreuses, de préférence des molécules cytotoxiques ou des molécules de la famille des inhibiteurs des protéines kinases.

De manière avantageuse, le procédé a consisté à faire exprimer la nanoluciférase aux cellules cibles.

Cette nanoluciférase est une protéine dérivée de la sous-unité de 19kDa d'une luciférase extraite de la crevette des grands fonds *Oplophorus gracilirostris*, la seconde sous-unité étant une protéine de 35 kDa (protéines décrites dans l'article de S. Inouye et al., Secretional luciferase of the luminous shrimp Oplophorus gracilirostris : cDNA cloning of a novel imidazopyrazinone luciferase, dans FEBS Letters, 481 (2000) 19-25). Cette nanoluciférase a été optimisée et commercialisée par la société Promega sous le nom de NanoLuc®, (et décrite dans M. Hall et al. Engineered luciferase reporter from a deep sea shrimp utilizing a novel imidazopyrazinone substrate dans ACS Chemical Biology 2012, 7, 1848-1857), dans le but d'améliorer les systèmes de gènes rapporteurs pour des systèmes i) d'étude des interactions protéine-protéine ou protéine-ligand ; ii) d'étude de stabilité protéique ; iii) de biosenseurs donneurs d'énergie vers une tierce molécule acceptrice (BRET) ; iv) d'imagerie in vivo ; v) d'études de la charge et de la réplication virales ; et vi) d'essais rapporteurs pour la signalisation cellulaire (études d'expression de gènes ou de suivi du métabolisme intracellulaire des protéines). Ont notamment été décrits à ce jour : l'utilisation intracellulaire en tant que gène rapporteur (voies de signalisation, liaison à un récepteur, par exemple), l'imagerie sur cellules/animaux vivants (marquage de cellules pour suivre leur cheminement biologique) ou le marquage de bactéries ou de virus pour le suivi d'infections; le couplage de la nanoluciférase à une protéine d'intérêt pour mesurer son internalisation ou sa sécrétion (Norisada et al., Biochem. Biophys. Res. Comm. 449:483, 2014). Ces systèmes ont en commun de coupler la séquence codante de la nanoluciférase à celle d'une autre protéine d'intérêt et/ou à la séquence d'un promoteur génétique activé spécifiquement par la voie de signalisation étudiée et/ou de se restreindre à une détection intracellulaire de la molécule.

Au contraire, l'approche des inventeurs a consisté à proposer un procédé dans lequel les cellules cibles sont transformées génétiquement afin d'exprimer transitoirement ou constitutivement ladite enzyme exogène (par exemple la nanoluciférase) sous une forme cytoplasmique et non sécrétée, plus particulièrement à faire exprimer ladite enzyme seule et sous forme libre dans le cytoplasme des cellules cibles et à mesurer uniquement et spécifiquement la quantité de molécules libérées dans le milieu extracellulaire suite à la lyse des cellules par des mécanismes tels que l'ADCC, la CDC, l'apoptose ou la lyse par un détergent.

A cet effet, l'étape de transformation génétique des cellules cibles comprend avantageusement l'introduction dans les dites cellules d'un vecteur d'expression portant la séquence codante de l'enzyme exogène et un promoteur de type constitutif permettant sa transcription dans une cellule, telle qu'une cellule eucaryote.

De préférence, le vecteur peut être un vecteur viral, ou un vecteur plasmidique, de préférence un vecteur plasmidique.

Avantageusement, le vecteur d'expression de l'enzyme exogène comprend un gène de résistance aux antibiotiques. Ce gène de résistance aux antibiotiques permet une sélection des cellules cibles eucaryotes ayant intégré le vecteur (cellules dites "transformées"). L'antibiotique de sélection est notamment capable d'éliminer les cellules eucaryotes ne portant pas le gène de résistance. Ce gène de résistance aux antibiotiques peut être, en particulier, un gène de résistance à la généticine (G418), la puromycine, la blasticidine, l'hygromycine B, l'acide mycophénolique ou la zéocine, de préférence un gène de résistancet à la puromycine.

De manière préférée, l'introduction dudit vecteur d'expression dans lesdites cellules est réalisée par infection par des particules virales portant le gène de l'enzyme exogène, lorsque le vecteur d'expression est un vecteur viral ou par des méthodes chimiques ou physiques lorsque le vecteur d'expression est un vecteur plasmidique. Lorsque le vecteur est un vecteur plasmidique, l'introduction dans les cellules cibles est avantageusement réalisée par électroporation.

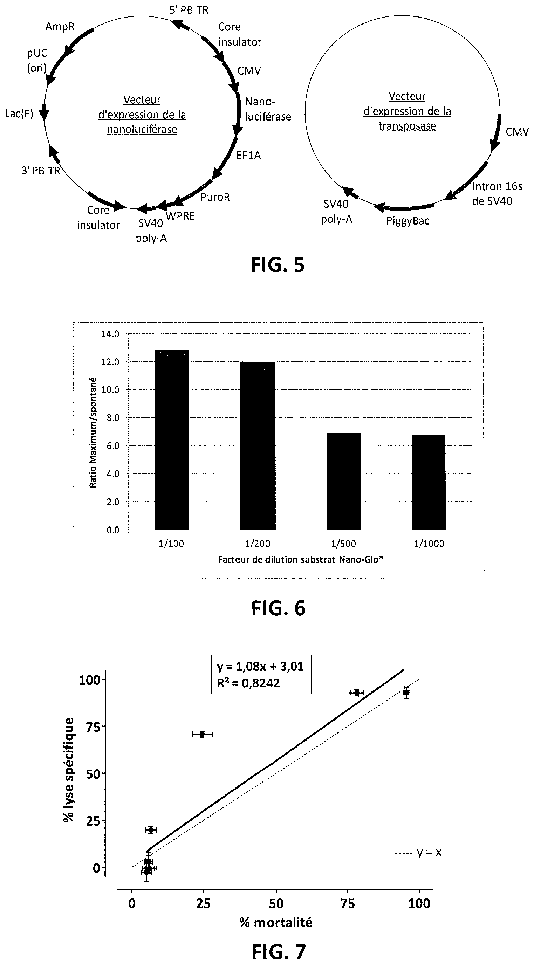

Ces différents éléments constitutifs sont assemblés selon les méthodes classiques de clonage par biologie moléculaire : utilisation d'enzymes de restriction couplées ou non à des réactions de polymérisation en chaîne (PCR) afin d'isoler les séquences d'ADN d'intérêt et re-ligature des différents constituants grâce à des enzymes de réparation ou de synthèse de l'ADN (ligases ou DNA polymérases, par exemple). On dispose alors d'un système permettant la transformation génétique d'une cellule cible pour qu'elle exprime l'enzyme exogène (par exemple la nanoluciférase) dans son compartiment cytoplasmique. Un exemple de système de transformation cellulaire basé sur la transposase piggyBac est donné en Figure 5.

Bien que cela ne soit pas indispensable à la mise en oeuvre de la présente invention, il est recommandé de procéder ensuite à une étape de clonage de ces mélanges cellulaires. Cela permet d'obtenir une lignée de cellules cibles exprimant la nanoluciférase et issue d'une seule cellule (ou clone), ce qui favorisera l'homogénéité et la reproductibilité des résultats du test de cytotoxicité.

Le procédé selon l'invention trouve une utilisation intéressante pour mesurer la cytotoxicité cellulaire dépendante d'anticorps (ADCC), mesurer la cytotoxicité dépendante du complément (CDC) et/ou mesurer l'apoptose.

La présente invention concerne également un kit pour la réalisation du procédé décrit ci-dessus, ou son utilisation, comportant :

- une lignée de cellules cibles exprimant l'enzyme exogène,

- des cellules effectrices cytotoxiques pour la réalisation d'un test d'ADCC et/ou une source de complément pour la réalisation d'un essai de CDC,

- un substrat activant ladite enzyme exogène pour produire une émission de lumière et les éventuelles solutions tampons associées,

- une notice explicative.

La présente invention va maintenant être décrite plus en détail à l'aide des exemples non limitatifs ci-après, mentionnés à titre d'illustration, en référence aux figures dans lesquelles :

La Figure 1 montre des résultats obtenus avec la méthode de l'art antérieur calcéine-AM (DELFIA) (exemple comparatif A);

La Figure 2 montre la comparaison entre la méthode au $^{51}$Cr et une méthode TR-FRET mettant en oeuvre deux anticorps, donneur et accepteur (exemple comparatif B) ;

La Figure 3 montre les résultats préliminaires d'une méthode TR-FRET mettant en oeuvre un anticorps donneur et la GFP (exemple comparatif C) ;

La Figure 4 montre la linéarité du signal luminescent généré par la nanoluciférase ainsi que la linéarité de la relation entre le nombre de cellules dans les puits après lyse au triton X-100 et l'intensité du signal ;

La Figure 5 montre la carte des plasmides utilisés pour transformer les cellules cibles ;

La Figure 6 illustre l'intensité du signal luminescent obtenu en fonction du facteur de dilution appliqué au substrat de la nanoluciférase ;

La Figure 7 présente la corrélation entre le pourcentage de mortalité mesuré par cytométrie en flux et le pourcentage de lyse spécifique obtenu par la méthode de la nanoluciférase ;

La Figure 8 décrit l'influence du temps de réaction sur la mesure de la lyse spécifique des cellules cibles dans un essai d'ADCC mettant en oeuvre la méthode ;

La Figure 9 montre la comparaison directe des méthodes nanoluciférase ("lumi.") et chrome-51 ("51Cr") sur le modèle Raji dans un essai d'ADCC (N=3 expériences, réalisées par 2 opérateurs différents : 2 expériences par l'opérateur n°1 et 1 expérience par l'opérateur n°2) ;

La Figure 10 montre la dispersion des données d'Emax et d'Emin des expériences d'ADCC décrites dans la Figure 9 ;

La Figure 11 montre la dispersion des données d'EC50 des trois expériences d'ADCC décrites dans la Figure 9 ;

La Figure 12 montre les résultats du calcul de potency pour les 3 expériences d'ADCC décrites dans la Figure 9 ;

La Figure 13 montre la comparaison directe des méthodes nanoluciférase ("lumi.") et chrome-51 ("51Cr") sur le modèle Raji dans un essai de CDC (N=3 expériences, réalisées par 2 opérateurs différents : 2 expériences par l'opérateur n°1 et 1 expérience par l'opérateur n°2) ;

La Figure 14 montre la dispersion des données d'Emax et d'Emin des expériences de CDC décrites dans la Figure 13 ;

La Figure 15 montre la dispersion des données d'EC50 des trois expériences de CDC décrites dans la Figure 13 ;

La Figure 16 montre les résultats du calcul de potency pour les 3 expériences de CDC décrites dans la Figure 13 ;

La Figure 17 montre la comparaison directe des méthodes nanoluciférase ("lumi.") et chrome-51 ("51Cr") sur le modèle SKOV3 dans un essai d'ADCC (N=3 expériences, réalisées par 2 opérateurs différents : 2 expériences par l'opérateur n°1 et 1 expérience par l'opérateur n°2) ;

La Figure 18 montre la dispersion des données d'Emax et d'Emin des expériences d'ADCC décrites dans la Figure 17 ;

La Figure 19 montre la dispersion des données d'EC50 des trois expériences d'ADCC décrites dans la Figure 17 ;

La Figure 20 montre les résultats du calcul de potency pour les trois expériences d'ADCC décrites dans la Figure 17 ;

La Figure 21 montre la comparaison directe des méthodes nanoluciférase et chrome-51 sur le modèle CHO-TNF-alpha dans un essai de CDC ;

La Figure 22 montre la dispersion des données d'Emax et d'Emin des expériences de CDC décrites dans la Figure 21 ;

La Figure 23 montre la dispersion des données d'EC50 des trois expériences de CDC décrites dans la Figure 21 ;

La Figure 24 montre les résultats du calcul de potency pour les 3 expériences de CDC décrites dans la Figure 21 ;

La Figure 25 montre les résultats du calcul de potency pour 3 expériences d'ADCC réalisées dans le modèle CHO-TNF-alpha ;

La Figure 26 résume les expériences d'évaluation de l'influence des interactions cellulaires sur la mesure de la lyse spécifique des cellules cibles dans un essai d'ADCC ou de CDC mettant en oeuvre la méthode nanoluciférase ;

La Figure 27 illustre la capacité de la méthode de libération de la nanoluciférase à mesurer la mort des cellules cibles induite par un traitement pro-apoptotique de type physique ;

La Figure 28 illustre la capacité de la méthode de libération de la nanoluciférase à mesurer la mort des cellules cibles induite par un traitement pro-apoptotique de type chimique.

**EXEMPLES**

Dans le procédé selon la présente invention décrit ci-après, les cellules cibles ont été perméabilisées par une série de chocs électriques (électroporation) en présence des deux plasmides d'intérêt présentés dans la Figure 5, l'un portant le transgène de la nanoluciférase et l'autre celui de l'enzyme permettant son intégration dans le génome, la transposase PiggyBac.
Légende de la figure 5 :

- AmpR : gène de résistance à l'ampicilline ;

- pUC (ori) : origine de réplication bactérienne ;

- Lac(F) : gène codant pour l'enzyme III lactose-spécifique de S. aureus ;

- 3'PB TR : Elément de recombinaison homologue 3' du système PiggyBac ;

- Core insulator : "chicken hypersensitivity site 4 core insulator" ;

- SV40-polyA : "Simian virus 40 Poly-Adenosine" ;

- WPRE : "Woodchuck hepatitis virus Posttranscriptional Regulatory Element" ;

- PuroR : gène de résistance à la puromycine ;

- EF1A : promoteur de l'EF1A ;

- Nanoluciférase : gène de la nanoluciférase ;

- CMV : promoteur du CMV

- 5'PB TR : Elément de recombinaison homologue 5' du système PiggyBac ;

- PiggyBac : gène de PiggyBac.

Plusieurs lignées de cellules cibles ont subi cette transformation génétique : la lignée Raji, la lignée SKOV3 et une lignée CHO exprimant le TNF-alpha humain.
Après transformation génétique des cellules cibles choisies, celles-ci sont mises en culture dans des conditions adéquates (généralement, pour des cellules de mammifères, la température est de 37$\pm$2°C dans une atmosphère humidifiée contenant entre 5 et 10% de CO2) dans un milieu de culture approprié au type cellulaire choisi contenant l'antibiotique de sélection dont le gène de résistance a été introduit dans le vecteur d'expression (la puromycine dans l'exemple présenté à la Figure 5). Cet antibiotique permet de sélectionner les cellules effectivement transformées en ne permettant pas la croissance des cellules pour lesquelles la transformation génétique n'a pas fonctionné. Une culture longue (quelques semaines) permet d'obtenir un mélange de cellules qui ont toutes intégré de manière stable le transgène dans leur ADN.

Bien que cela ne soit pas indispensable à la mise en oeuvre de la présente invention, il est recommandé de procéder ensuite à une étape de clonage de ces mélanges cellulaires. Cela permet d'obtenir une lignée de cellules cibles exprimant la nanoluciférase et issue d'une seule cellule (ou clone), ce qui favorisera l'homogénéité et la reproductibilité des résultats du test de cytotoxicité.

Afin de garantir la stabilité et la pérennité de l'approvisionnement en cellules cibles transformées, il est préférable de produire une banque de ces cellules s dans des conditions permettant leur croissance statiquement ou sous agitation dans des conteneurs adaptés. Par exemple, pour produire les banques cellulaires ayant permis de confirmer la validité du procédé de la présente invention, les cellules ont été cultivées statiquement en flasques traitées pour culture cellulaire, à $37 \pm 2°C$ sous atmosphère humide à $5 \pm 1\%$ de CO2 et en milieu adapté : RPMI-1640 contenant 10% de sérum de veau foetal (SVF) et 0,25g/mL de puromycine pour la lignée Raji ; en milieu McCoy 5A, 10% SVF et 5$\mu$g/mL de puromycine pour les cellules SKOV3 ; en milieu F12-Ham, 10% SVF, 1mg/mL G418 et 20$\mu$g/mL puromycine pour les cellules CHO. Après croissance d'une quantité suffisante de cellules, celles-ci sont réparties dans des contenants adaptés et congelées selon les techniques habituelles aux cellules eucaryotes pour leur cryopréservation.

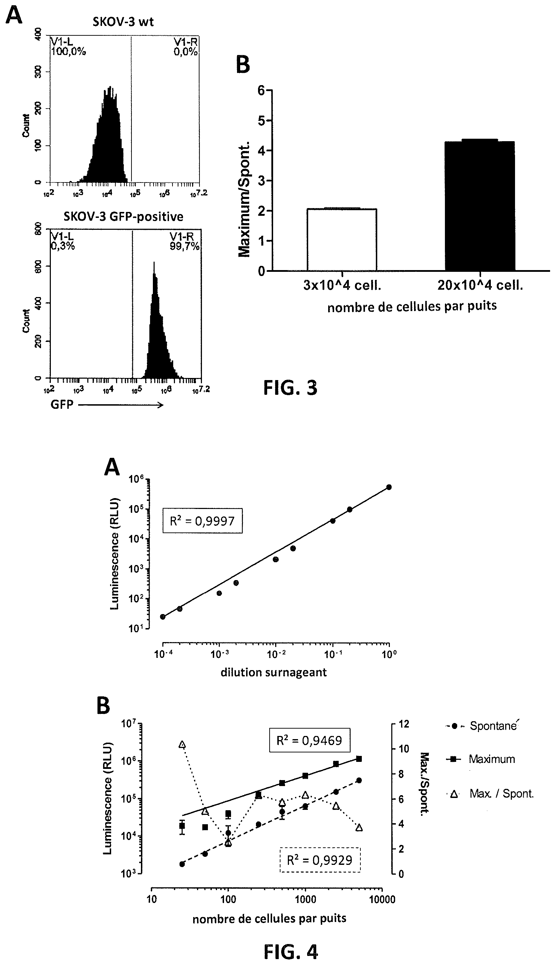

La Figure 4 illustre le type de signal qu'il est possible d'obtenir avec de telles cellules cibles (les cellules CHO exprimant le TNF-alpha dans cet exemple) exprimant la nanoluciférase après transformation génétique. Ces résultats montrent d'une part que le signal généré par la nanoluciférase libre en solution (après production par les cellules) est parfaitement linéaire entre $10^1$ et $10^6$ unités relatives de luminescence (RLU). Ils montrent d'autre part que le rapport entre signal maximum (après lyse des cellules par un détergent) et libération spontanée est toujours au moins égal à 2 dans les conditions testées, et se stabilise autour de 6 lorsque le nombre de cellules par puits est compris entre 250 et 5000 cellules. La quantité de cellules cibles utilisées dans les essais présentés ci-après est donc comprise dans cette fourchette. Les conditions d'utilisation et la sensibilité de ce système permettent donc une grande souplesse dans la mise en oeuvre du test. Le très faible nombre de cellules nécessaires à l'obtention d'un signal satisfaisant est particulièrement remarquable lorsqu'il est comparé à la quantité de cellules que nécessitent les techniques de l'art antérieur (voir Tableau 1 comparant les performances des différentes méthodes de mesure spécifiques de la lyse directe [art antérieur et différents exemples de l'invention décrits ci-après avec la nanoluciférase]).

**Tableau 1**

| Méthode | Ratio Signal/ Bruit | Quantité nécessaire de cellules | Radio-Actif | Sensibilité | Reproducti bilité | Capacité analyse haut débit |
|---|---|---|---|---|---|---|
| Chrome $^{51}$Cr | 5-12 | 2500 à 10000 | + | + | + | + |
| Calcéine-AM | 2.5 | 2500 à 10000 | - | -/+ | -/+ | + |
| $Eu^{3+}/Tb^{3+}$ | 1-3 | 5000 à 10000 | - | -/+ | -/+ | + |
| Cytométrie en flux | NA | 1000 à 5000 | - | + | -/+ | |
| Beta-galactosidase | $\approx$30 | $2x10^5$ | - | -/+ | Pas de données disponibles | + |
| Nanoluciférase (présente invention) | 4-12 | 250 à 5000 | - | + | + | + |

Légende." NA" : non applicable ; "+" : oui ; "-" : non ; "-/+" : faible.

Parmi les méthodes de l'Art Antérieur : la méthode actuelle de référence est la méthode au chrome $^{51}$Cr, les méthodes calcéine-AM et $Eu^{3+}/Tb^{3+}$ sont peu sensibles et variables selon le type cellulaire, et la méthode à la béta-galactosidase sous-estime de 30-40 % la proportion de cellules mortes.

**Réalisation du test ou essai de cytotoxicité selon l'invention :**

Les cellules cibles préparées comme décrit précédemment sont mises sous la forme d'une suspension homogène et comptées par toute méthode adéquate. La quantité de cellules nécessaire à la réalisation de l'essai est déposée dans un tube à centrifugation et le milieu de culture est éliminé par centrifugation entre 1 et 30 minutes à une accélération de 100 g à 1500 g. De préférence, cette centrifugation est classiquement réalisée entre 120 g et 600 g pendant 5 à 10 minutes, le milieu de culture surnageant est éliminé et les cellules sont reprises dans du milieu de culture neuf (de préférence ne contenant pas le ou les antibiotiques de sélection) à la concentration souhaitée pour la réalisation de l'essai final.

L'essai de cytotoxicité peut être réalisé dans toute sorte de contenants (tubes ou plaques) permettant de maintenir des cellules eucaryotes vivantes pendant quelques heures, notamment des plaques multipuits à 96 ou 384 puits à fonds plats, coniques ou ronds. Chaque puits de test contient un mélange incluant la quantité de cellules cibles choisie, le produit dont l'action thérapeutique/cytolytique est à tester et, s'il y a lieu, un système effecteur complémentaire tel que des cellules cytotoxiques (dans le cas d'un essai de type ADCC ou réaction lymphocytaire mixte par exemple) ou une source de complément (dans le cas d'un essai de CDC ou assimilé). Sur la base des données présentées dans la Figure 4, la quantité de cellules cibles par puits sera comprise entre 10 et 10000, de préférence entre 200 et 3000.

La substance active est testée en utilisant différentes concentrations de celle-ci, une seule concentration par puits. Lorsqu'un système effecteur (de type cellules effectrices ou source de complément) est nécessaire, la substance à tester peut être ajoutée aux cellules cibles soit simultanément soit avant l'ajout du système effecteur, en respectant un temps de pré-incubation variable, de préférence compris entre 5 et 60 minutes. Plusieurs tubes ou puits pourront être utilisés avec des conditions strictement similaires afin de réaliser plusieurs "réplicats".

Dans le cas d'un essai d'ADCC ou de réaction lymphocytaire mixte, le ratio entre les quantités de cellules effectrices et de cellules cibles est de préférence compris entre 0,1 et 200. Les cellules effectrices peuvent être n'importe quelles cellules capables d'exercer une activité cytotoxique envers la lignée cible choisie, telles que des cellules NK, des lymphocytes T, des monocytes, des macrophages ou des polynucléaires, primaires ou immortalisées, non modifiées ou transformées génétiquement. Cette activité cytotoxique pourra être naturelle (c'est-à-dire se déclencher directement lorsque cellules cibles et cellules effectrices entrent en contact) ou induite par une substance activatrice, qui pourra être ou non la substance à tester (solutions d'anticorps monoclonaux, polyclonaux ou molécules apparentées, molécules pro-cytotoxiques, cytokines, hormones, neurotransmetteurs, etc.). Les cellules effectrices, en culture ou provenant d'une ampoule issue d'une banque cellulaire décongelée extemporanément, sont donc comptées par toute méthode adéquate. La quantité de cellules nécessaire à la réalisation de l'essai est alors déposée dans un tube à centrifugation et le milieu de culture est éliminé par centrifugation entre 1 et 30 minutes à une accélération de 100 g à 1500 g, de préférence entre 120 g et 600 g pendant 5 à 10 minutes. Le milieu de culture surnageant est ensuite éliminé et les cellules sont reprises dans du milieu de culture neuf (de préférence le même que pour les cellules cibles) à la concentration souhaitée pour la réalisation de l'essai final. Cette concentration dépend du nombre de cellules cibles utilisé par condition et du ratio effecteur/cible choisi.

Dans le cas où l'activation du complément est recherchée, une source de complément est ajoutée dans les tubes ou puits. Cette source de complément peut être un sérum animal (par exemple issu de bovins, d'ovins, de caprins, de rongeurs, de lapins, de singes ou d'êtres humains) ou être constitué d'un mélange des différentes molécules constitutives du système du complément purifiées et/ou recombinantes. Le système du complément étant très bien conservé au cours de l'Evolution, l'espèce de laquelle provient la source de complément a peu d'importance. Ce système expérimental est également approprié pour tester l'effet cytolytique du sérum en lui-même (sans l'ajout d'anticorps exogène), par exemple dans les processus de criblages de sérums auto-immuns ou vaccinaux. Globalement, la proportion de sérum ou de la source de complément peut représenter de 0,1% à 100% du volume réactionnel total.

Alternativement, le test peut être réalisé en utilisant une concentration unique de la substance cytotoxique à tester, en faisant varier la quantité de système effecteur dans les puits (ratio effecteur/cible ou quantité de complément par exemple), ou un mélange des 2 approches. Quoi qu'il en soit, le principe de l'essai reste le même.

Après avoir mélangé dans les tubes ou les puits les quantités voulues de cellules cibles, de substance(s) à tester et/ou du (des) système(s) effecteur(s), les tubes ou plaques de culture sont incubés entre 34°C et 40°C, de préférence à 37°C±1°C, dans une étuve ou un incubateur. Le temps d'incubation peut être variable en fonction du processus étudié. Dans le cas de phénomènes tels que l'ADCC ou la CDC, le temps d'incubation sera généralement compris entre 1 heure et 8 heures, et de préférence entre 2 heures et 5 heures. Dans le cas de phénomènes d'apoptose ou d'effets cytotoxiques à long terme, la durée de l'essai peut s'étendre de 8 heures à 72h, de préférence entre 24h et 48h.

A la fin de l'essai, le surnageant de chaque puits est récupéré. De préférence, et pour s'assurer que des cellules ne seront pas prélevées, les plaques ou les tubes contenant les cellules sont centrifugés entre 10 s et 30 min à une accélération de 100 g à 1500 g, de préférence entre 30 et 120 s à une vitesse comprise entre 200 g et 1000 g. Alternativement, une étape intermédiaire de prélèvement du surnageant directement dans les puits ou les tubes réactionnels, suivie du transfert de ce surnageant dans une plaque ou des tubes neuf(s) et de sa centrifugation aux conditions ci-dessus peut être réalisée. Le volume de surnageant nécessaire à la lecture est ensuite transféré dans une plaque appropriée à la lecture dans un luminomètre, c'est-à-dire opaque et de couleur blanche ou noire, et mélangé à un substrat de la nanoluciférase. Ces substrats sont de la famille de la coelentérazine et de ses analogues, et de préférence de type furimazine disponible dans le commerce auprès de la société Promega sous la référence produit "Nano-Glo® Luciferase Assay Substrate". En fonction du type de plaque utilisé, le surnageant est mélangé au substrat pour un volume final de réaction de 25 μL à 340 μL en plaques 96 puits standard, de 15 μL à 175 μL pour des plaques 96 puits format "demi-puits", de 15 μL à 110 μL pour des plaques 384 puits standards, de 4 μL à 25 μL pour des plaques 384 puits "petit volume", de 3 μL à 10 μL pour les plaques 1536 puits.

En fonction des recommandations du fournisseur et des résultats présentés dans la Figure 6, le substrat "Nano-Glo® Luciferase Assay Substrate" est dilué par mélange au surnageant (lui-même pur ou dilué en fonction de l'intensité attendue du signal et de façon dépendante du modèle cellulaire) dans un rapport compris entre 1/50 (1 volume de substrat pour 50 volumes de surnageant) et 1/1000, et de préférence compris entre 1/100 et 1/200. Après un temps d'incubation minimum de 3 minutes, la plaque est lue dans un luminomètre (par exemple modèle Mithras LB 940). Le temps d'acquisition des signaux est de l'ordre de 0,05 seconde. Comme démontré dans les exemples présentés ci-après, l'intensité du signal lumineux est proportionnelle à la lyse des cellules cibles. Le signal généré est fort, aboutissant à une méthode sensible, même avec de faibles quantités de cellules cibles (à partir de 250 cellules par puits). Les exemples ci-après montrent que le procédé selon l'invention basé sur la mesure de la libération de nanoluciférase présente des caractéristiques et des performances tout à fait comparables à celles du test actuel de référence basé sur la libération de $^{51}$Cr pour la détection et la mesure de la mort cellulaire (voir Tableau 2 dans l'exemple 10). Il présente en revanche le très gros avantage d'éviter l'utilisation d'éléments radioactifs, et donc toutes les contraintes et tous les risques liés à cette utilisation, tout en permettant un déroulé d'expérience plus rapide car il ne nécessite pas de temps de marquage des cellules cibles. Le principe de lecture par luminométrie constitue en outre une méthode simple et économique, très répandue dans les laboratoires et ne nécessitant pas l'achat d'équipement coûteux. Enfin, son mécanisme de fonctionnement est tout à fait similaire à celui du test au chrome et la mesure est spécifique de la mort des cellules cibles, même lorsque celles-ci sont mélangées à d'autres types de cellules dont la mort ne doit pas être mesurée.

L'ensemble de ces données démontre que le test de cytotoxicité basé sur la libération de nanoluciférase présente toutes les caractéristiques requises pour devenir un test de référence, en particulier au niveau industriel, pour tout essai nécessitant de mesurer spécifiquement la mort effective d'une population cellulaire donnée. De façon non restreinte, ces tests pourraient évaluer l'effet cytotoxique de polluants ou de contaminants, de facteurs environnementaux et de médicaments ou drogues cytotoxiques produites par une synthèse chimique ou par un système biologique et de tout autre élément toxique pour une population cellulaire donnée.

### Exemple comparatif A :

Trois lignées cellulaires différentes ont été marquées selon la méthode du kit DELFIA BATDA (Perkin-Elmer), strictement selon les recommandations du fabricant. 10000 cellules par puits (3 puits par condition) sont ensuite incubées 2 heures à 37°C dans du milieu de culture seul (RPMI [Roswel Park Memorial Institute] contenant 10% de sérum de veau foetal [SVF]) pour déterminer la libération spontanée ou dans du milieu de culture contenant 1% de détergent Triton X-100 pour déterminer la libération maximale (volume final des puits = 200 μL). Le bruit de fond est déterminé sur du milieu de culture seul, sans cellule ni triton X-100. A la fin de l'incubation, 20 μL de surnageant sont transférés dans des plaques spécifiques (fournies dans le kit) et 200 μL de solution d'europium sont ajoutés. Après 15 minutes d'incubation, les signaux sont lus par méthode TRF sur un lecteur adapté (Mithras LB 940, Berthold Technologies, Thoiry, France). Sur la figure 1A sont présentées les valeurs absolues mesurées pour les différents paramètres en comptes par seconde pour les différentes lignées. La figure 1B représente les ratios entre le signal maximal et le bruit de fond (barres blanches) d'une part ou le signal spontané (barres noires) d'autre part. Cette figure montre les résultats d'une expérience représentative d'environ dix expériences au cours desquelles différentes approches d'optimisation de l'intensité du signal ont été tentées sans succès, avec des ratios signal/bruit toujours inférieurs à 3.

### Exemple comparatif B :

Une première molécule reportrice artificielle a été testée par les inventeurs, combinant deux motifs antigéniques spécifiques distincts (appelés "flags"), chacun reconnus par un anticorps portant, pour l'un, un lanthanide donneur d'énergie et, pour l'autre, un accepteur d'énergie. L'ensemble représente une protéine d'un poids de 47 kDa. La présence de la molécule libérée est détecté par ajout des deux anticorps simultanément dans le surnageant. Si de la molécule libre est présente, les deux anticorps s'y fixent, se retrouvant ainsi à proximité directe l'un de l'autre, ce qui permet le transfert d'énergie et l'émission d'un signal de type TR-FRET.

Dans l'exemple, des cellules cibles SKBR3 exprimant cette molécule reportrice sont incubées 4 heures à 37°C en plaque à 96 puits en présence de concentrations croissantes (indiquées en abscisses) de trastuzumab et de cellules cytotoxiques (lymphocytes T exprimant le récepteur CD16). A la fin des 4 heures d'incubation, les anticorps de révélation sont ajoutés au milieu et le signal TR-FRET est lu en fluorimétrie sur un lecteur Mithras LB 940 (Berthold) (décalage de lecture de 300 μs, ratio 665/620 nm).

En parallèle, une fraction des mêmes cellules SKBR3 exprimant la molécule reportrice est marquée avec du

$^{51}$Cr (100 µCi/10$^6$ cellules). Le reste des conditions expérimentales est strictement identique : nombre de cellules cibles (3000) et de cellules effectrices (30000) par puits, milieu de culture (RPMI et 10% de sérum de veau foetal), deux puits en duplicats pour chaque concentration testée, 4 heures d'incubation à 37°C. A la fin de l'incubation, le surnageant est récupéré, transféré dans des plaques à scintillation (Lumaplate, Perkin-Elmer) et lues dans un compteur à scintillation (Microbeta Jet, Perkin-Elmer).

Les résultats, présentés en figure 2, sont exprimés en pourcentage de lyse spécifique, calculés selon la formule :

$$\frac{Signal\ (X) - Signal\ spontané}{Signal\ maximum - Signal\ spontané}$$

dans laquelle "Signal (X)" est le signal obtenu pour un puits X donné, "Signal maximum" est le signal obtenu lorsque les cellules sont lysées par 0,75 % de triton X-100 et "Signal spontané" est le signal obtenu lorsque les cellules sont simplement incubées en présence de milieu de culture seul (sans anticorps ni cellules effectrices). Le graphique montre en ordonnées la lyse spécifique moyenne ± écart type pour chaque concentration testée selon les deux méthodes, en $^{51}$Cr (ronds noirs et trait plein) ou TR-FRET (triangles noirs et traits pointillés). Les résultats présentés sur la figure 2 portent sur une expérience représentative de deux.

Ces résultats montrent l'inefficacité de la molécule reportrice à être représentative de la lyse des cellules cible telle qu'observée par la méthode au $^{51}$Cr. Les contrôles positifs (non montrés) étant par ailleurs satisfaisants, la méthode de détection en elle-même n'est pas à mettre en cause. La molécule reportrice est donc probablement retenue dans le cytoplasme des cellules cibles et n'est pas libérée au cours de la lyse. La taille importante de la molécule (47kDa) est l'hypothèse privilégiée pour expliquer cette rétention cytoplasmique.

**Exemple comparatif C :**

Une autre protéine reportrice, une GFP ("Green Fluorescent Protein") recombinante de taille plus petite que celle présentée dans l'exemple comparatif B ci-dessus (approximativement 27 kDa), et dont la présence dans le surnageant peut être mesurée par l'emploi d'un anticorps couplé à un lanthanide (qui effectue un transfert d'énergie TR-FRET vers la GFP lorsqu'il est fixé à celle-ci), a été testée dans cet exemple comparatif.

Une lignée cible de cellules SKOV3 transformée génétiquement pour exprimer la GFP recombinante a été utilisée pour ces essais, dont les résultats sont présentés sur la figure 3. La partie gauche (A) de la figure 3 montre l'expression de la GFP mesurée par cytométrie en flux sur les cellules SKOV3 sauvages (wt) non transformées, en haut, ou par les cellules SKOV3 transformées (GFP-positives), en bas. L'expression de la GFP par les cellules transformées correspond approximativement à 1,5-Log, ce qui est un niveau satisfaisant.

La partie droite de la figure 3 (B) représente l'essai dans lequel 30000 ou 200000 cellules ont été déposées en triplicat dans des puits d'une plaque à 96 puits et incubés en présence de milieu de culture seul (RPMI contenant 10% de SVF) (libération spontanée) ou de milieu de culture plus 0,75% de triton X-100 (libération maximale). En fin d'incubation, le surnageant est prélevé et un anticorps anti-GFP couplé à un lanthanide (Tb) est ajouté. Le signal mesuré (lecteur Mithras LB 940, Berthold Technologies) est un signal TR-FRET du lanthanide vers la GFP (décalage de lecture = 300 µs, ratio 520/620 nm). La figure 3B représente le ratio entre le signal maximum et le signal spontané pour chacune des deux quantités de cellules, et est représentatif d'une expérience parmi deux.

Les résultats montrent que, même avec une quantité importante de cellules cibles par puits (3x10$^4$), le ratio maximum sur spontané n'est que de 2, et qu'il faut une quantité considérable de cellules (2x10$^5$) pour commencer à observer un ratio intéressant (de l'ordre de 4). Cette méthode est donc bien loin des performances du $^{51}$Cr en matière de sensibilité (comme évoqué précédemment, celui-ci se situe entre 8 et 12 avec 3000 cellules par puits). Ici encore, la taille de la GFP et/ou les mécanismes de sa libération au cours de la lyse des cellules ne sont pas compatibles avec une mesure efficace de la mort cellulaire.

**Exemple 1 :** montre la linéarité du signal luminescent et l'influence de la quantité de cellules sur ce signal.

La luminescence d'un surnageant contenant de la nanoluciférase a été mesurée, après lyse au triton X-100, dilué en série entre 1/1 et 1/10000. 25µL de chaque dilution sont mélangés à 25 µL de substrat Nano-Glo® (Promega) dans une plaque 96 puits "demi-puits" avant lecture au luminomètre (Mithras LB 940, Berthold Technologies, temps d'acquisition 0,05 s). Une régression linéaire des valeurs obtenues est réalisée (voir figure 4A : intensité du signal luminescent obtenu, exprimé en RLU (Relative Luminescence Unit) en fonction de la dilution) On constate d'une part que ce signal est linéaire (coefficient de régression R$^2$ = 0,9997) sur toute la gamme de mesure et s'étend environ de 10 à 10$^6$ unités arbitraires de luminescence sur le lecteur utilisé.

D'autre part, la linéarité des signaux de libération spontanée et maximum de cellules CHO-K1 exprimant la nanoluciférase a été évaluée en incubant 4 h des quantités variables de cellules cibles (10000, 5000, 2500, 1000, 500, 250, 100, 50 et 25 cellules par puits) en présence de milieu seul (spontané) ou de 0,25 % de triton X-100 (maximum). 25 $\mu$L de surnageant sont alors prélevés et mélangés à 25 $\mu$L de substrat Nano-Glo® avant lecture au luminomètre (temps d'acquisition 0,05 s). Les résultats sont présentés sur la figure 4B. L'axe des abscisses montre le nombre de cellules par puits et l'axe des ordonnées de gauche représente le signal luminescent obtenu dans les conditions spontané (ronds noirs) et maximum (carrés noirs). Une régression linéaire est réalisée pour chacune de ces deux séries de points et représentée sur le graphe (respectivement ligne à pointillés larges et trait continu), ainsi que le facteur de corrélation ($R^2$) associé, qui montre une très bonne relation linéaire entre ces paramètres. Pour chaque quantité de cellules, le ratio entre signal maximum et signal spontané est calculé et représenté par un triangle blanc sur l'axe des ordonnées de droite, chaque point étant relié par une ligne à pointillés fins pour faciliter la lecture..

L'ensemble de ces données démontre la faisabilité de cette méthode, qui s'avère sensible (25 cellules par puits suffisent à avoir un signal satisfaisant), linéaire sur une large gamme de quantité de cellules et de luminescence (tous les facteurs de régression analysés sont supérieurs à 0,94) et souple d'emploi (le ratio [libération maximale]/[libération spontanée] reste constant entre 250 et 2500 cellules par puits).

**Exemple 2** : montre la relation linéaire entre l'intensité du signal luminescent et le nombre de cellules effectivement mortes au cours d'un essai de cytotoxicité.

Un essai de lyse par le complément a été réalisé sur des cellules Raji (exprimant la nanoluciférase) en présence d'un anticorps thérapeutique anti-CD20 disponible dans le commerce, le MabThera® (INN : rituximab).

Ces cellules Raji exprimant la nanoluciférase sont incubées 4h à 37°C en plaques 96 puits à fonds plats (3000 cellules par puits) en milieu RPMI-1640, de quantités variables d'anticorps anti-CD20 (MabThera®, Roche, gamme de 9 concentrations de 1093,5 à 0,167 ng/mL par pas de dilution de 1/3) et de 1CH$_{50}$ ("complement haemolytic 50", unité de mesure usuelle de l'activité du complément) de complément de cobaye (Sigma-Aldrich). Des conditions contrôles sont également réalisées permettant de mesurer la libération spontanée (3000 cellules cibles en RPMI-1640, 1CH$_{50}$ de complément de cobaye et 1093,5 ng/mL d'un anticorps ne se fixant pas aux cellules Raji, l'Herceptin®) et la libération maximum de nanoluciférase en présence de 0,01% de triton X-100. Chaque condition opératoire est réalisée en triplicat. A la fin de l'incubation, 25 $\mu$L de surnageant sont récupérés dans chaque puits, mélangés à 25 $\mu$L de substrat Nano-Glo® dilué au 1/50 en PBS, incubés 3 min et lus au luminomètre.

Le pourcentage de lyse spécifique obtenu pour chaque concentration X est calculé à partir des mesures de luminescence (en RLU) selon la formule : (RLU(X)- RLU(spontané))/(RLU(max)-RLU(spontané)) $\times$100.

Après prélèvement du surnageant, le reste des cellules est remis en suspension, mélangé extemporanément avec 0,25 $\mu$M final du marqueur vital TO-PRO-3 (Life Technologies) et analysé par cytométrie en flux afin de déterminer le pourcentage de mortalité (c'est-à-dire le pourcentage de cellules marquées au TO-PRO-3). La figure 7 présente les résultats obtenus pour chacune des concentrations de MabThera®. Chaque point représente la moyenne des triplicats, les barres verticales et horizontales représentant l'écart type. La droite théorique y = x est représentée par une ligne pointillée. Les résultats montrent que la droite de régression a une pente de 1,08$\pm$0,10, une ordonnée à l'origine de 3,01$\pm$4,21 et un coefficient de régression linéaire de 0,8242. Ces résultats montrent une relation proportionnelle et linéaire satisfaisante entre les deux méthodes de mesure de la cytolyse, ce qui permet de confirmer que le phénomène de lyse mesurée par la méthode de la nanoluciférase se traduit bien fonctionnellement par une mort effective des cellules au niveau biologique.

**Exemple 3** :

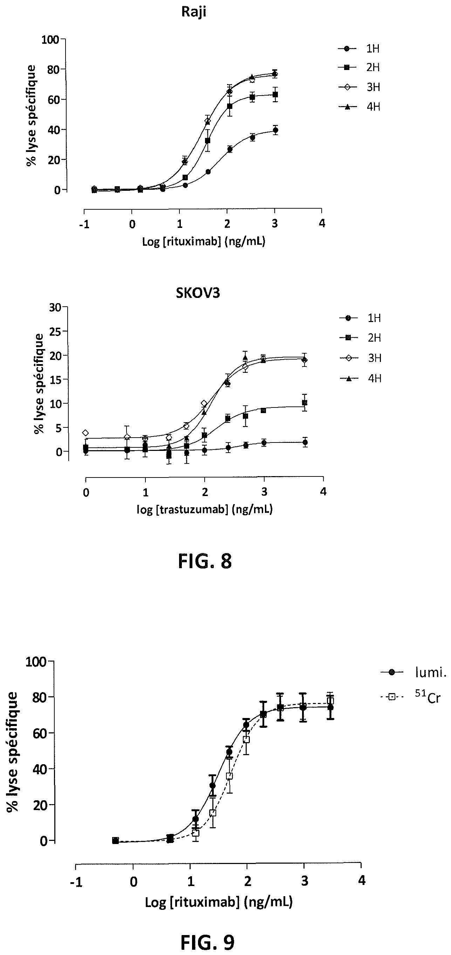

La Figure 8, dans laquelle la lyse spécifique de cellules adhérentes (SKOV3) ou non adhérentes (Raji) exprimant la nanoluciférase a été mesurée dans un essai d'ADCC selon la présente invention, montre le caractère dynamique de ladite méthode.

Des cellules cibles non adhérentes (Raji) ou adhérentes (SKOV3) transformées pour exprimer stablement la nanoluciférase ont été incubées (à 37°C dans une atmosphère humidifiée à 5% de CO2), en plaques 96 puits à fonds plats traités pour culture cellulaire (3000 cellules cibles par puits), en présence de concentrations croissantes (dont les logarithmes sont indiqués en abscisses) de l'anticorps correspondant (rituximab [MabThera®] ou trastuzumab [Herceptin®], respectivement) et de cellules effectrices cytotoxiques (lymphocytes T exprimant CD16, 30000 cellules effectrices par puits) dans du RPMI-1640 supplémenté par 5% de SVF. Chaque gamme est déposée 4 fois (pour les 4 durées d'incubation testées) avec 3 puits par condition (triplicat). Après 1 (ronds noirs), 2 (carrés noirs), 3 (losanges blancs) ou 4 (triangles noirs) heures d'incubation, 25$\mu$L de surnageant sont récupérés dans les puits correspondants, transférés dans une plaque "demi-puits" blanche, mélangés à 25 $\mu$L de substrat NanoGlo®dilué au 1/50 en D-PBS, incubés 3 minutes et lus sur un luminomètre Mithras LB940. Le pourcentage de lyse spécifique obtenu pour chaque concentration X est calculé à partir des mesures de luminescence (en RLU) selon la formule (RLU(X)- RLU(spontané))/(RLU(max)- RLU(spontané)) x100, les conditions maximum et spontané étant obtenues par incubation des mêmes cellules dans un milieu contenant un anticorps irrelevant (3000 ng/mL de trastuzumab ou 5000 ng/mL de rituximab, respectivement, pour constituer la mesure de la libération spontanée) et 0,01% de triton X-100 (libération maximum). Les pourcentages de lyse spécifiques sont représentés en ordonnées, chaque point représentant la moyenne du triplicat et les barres verticales représentent son écart-type. Les données ont ensuite été modélisées à l'aide d'une régression sigmoidale à 4 paramètres en utilisant le logiciel GraphPad Prism.

On observe une augmentation graduelle de la quantité de cellules lysées, concomitante d'une part avec l'augmentation de la concentration en agent cytolytique (l'anticorps thérapeutique) et d'autre part avec le temps d'incubation. Dans les deux modèles, 1 et 2 heures sont des temps trop courts pour observer l'effet lytique maximal alors que 3 et 4 heures montrent une saturation du phénomène de cytotoxicité. Dans ce dernier cas, un temps d'incubation supérieur à 3h ne permet pas d'obtenir une lyse plus élevée des cellules cibles. La présente méthode est donc en outre capable d'évaluer les aspects cinétiques de la cytolyse.

**Exemple 4** :

Afin d'évaluer leurs performances dans des conditions strictement comparables, les méthodes du $^{51}$Cr et de la nanoluciférase ont été comparées en parallèle dans un même essai d'ADCC spécifique de la molécule CD20, répété 3 fois de façon indépendante (un des essais étant réalisé par un second opérateur). Les mêmes cellules Raji exprimant la nanoluciférase ont été marquées ou non au $^{51}$Cr et utilisées en parallèle dans l'essai.

Des cellules cibles Raji transformées pour exprimer stablement la nanoluciférase ont été incubées ou non 1 heure dans une solution saline de $Na_2CrO_4$ équivalant à 100 $\mu$Ci de $^{51}$Cr par million de cellules, puis lavées 4 fois par du RPMI-1640, 10% SVF et comptées. Les cellules (3000 par puits), marquées ou non au $^{51}$Cr, ont ensuite été incubées à 37°C dans une atmosphère humidifiée à 5% de $CO_2$ en plaques 96 puits à fonds plats traitées pour la culture cellulaire, en présence de concentrations croissantes de l'anticorps rituximab (MabThera®) et de cellules effectrices cytotoxiques (lymphocytes T exprimant CD16, 30000 cellules par puits) dans du RPMI-1640 supplémenté par 5% de SVF.

Après 4 heures d'incubation, le pourcentage de lyse spécifique est mesuré selon la méthode correspondante (libération de $^{51}$Cr ou de nanoluciférase selon que les cellules aient été marquées au chrome ou non) : 25 $\mu$L de surnageant des cellules marquées au $^{51}$Cr sont récupérés et déposés sur une plaque Lumaplate® (Perkin Elmer). Au même moment, pour les cellules non marquées au $^{51}$Cr, 25$\mu$L de surnageant sont récupérés dans chaque puits, transférés en plaques 96 "demi-puits" blanches, mélangés à 25 $\mu$L de substrat NanoGlo®dilué au 1/50 en D-PBS, incubés 3 minutes et lus sur un luminomètre Mithras LB940 (Berthold Technologies). Après une nuit de séchage, la radioactivité des surnageants déposés en Lumaplate® est mesurée dans un compteur Microbeta-Jet (Perkin Elmer) et exprimée en CCPM ("corrected count per minute") après normalisation des détecteurs.

Le pourcentage de lyse spécifique obtenu pour chaque concentration X est calculé de la même façon pour les deux types de méthodes, à partir des mesures de radioactivité (en CCPM) ou de luminescence (en RLU) selon la formule

$$\frac{CCPM/RLU[X] - CCPM/RLU[spontané]}{CCPM/RLU[max] - CCPM/RLU[spontané]} \times 100.$$

Dans les deux cas, les conditions maximum et spontané ont été obtenues par incubation des mêmes cellules dans un milieu contenant 3000 ng/mL de trastuzumab en remplacement du rituximab (libération spontanée) et 0,01% de triton X-100 (dans le cas de la libération maximum uniquement). Les pourcentages de lyse spécifiques sont représentés sur la figure 9 en ordonnées, chaque point représentant la moyenne des neuf déterminations (3 réplicats dans 3 expériences indépendantes) et les barres verticales représentent les écarts-types. Les données ont été modélisées à l'aide d'une régression sigmoidale à 4 paramètres en utilisant le logiciel GraphPad Prism.

Cette modélisation permet de calculer quatre grandeurs caractéristiques de la courbe : les pourcentages de lyse minimale (Emin) et maximale (Emax), la pente de la partie linéaire et la concentration d'anticorps nécessaire pour obtenir 50% de l'Emax ($EC_{50}$). La capacité des deux méthodes à détecter des échantillons dont l'activité ADCC serait légèrement modifiée par rapport à un échantillon de référence (ce qui constitue la base d'une mesure de potency) a été évaluée grâce à l'utilisation de gammes d'anticorps dont les concentrations sont décalés de 80% ou de 125% par rapport à la gamme de référence (dite "100%"). La potency des gammes diluées à X% est calculée selon la formule : $(EC_{50}[100\%])/(EC_{50}[X\%]) \times 100$. Cette approche permet également d'évaluer la justesse de la méthode selon les critères de l'ICH-Q2(R1).

Les résultats généraux de ces trois expériences sont illustrés sur la Figure 9, qui montre les pourcentages de lyse spécifique obtenus (moyenne $\pm$ écart-type des 3 expériences) et les modélisations résultantes pour la gamme 100%. Ils montrent une très bonne reproductibilité des deux méthodes (les écart-types pour une même condition sont peu élevés) ainsi qu'un profil de résultats très similaire entre les deux méthodes (les deux courbes sont quasiment superposables).

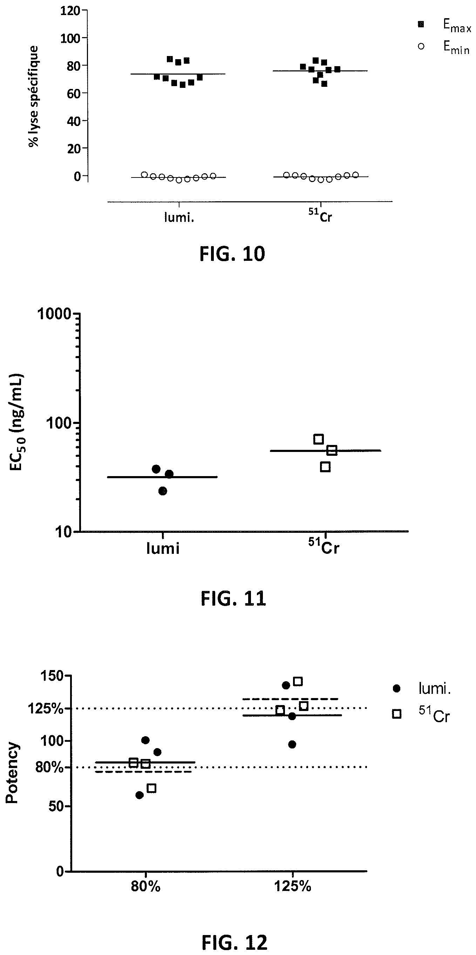

Pour aller plus loin dans l'analyse, la dispersion des Emin et Emax calculés pour chaque série de données, pour les 3 gammes et dans les 3 expériences a été analysée. Les résultats sont présentés sur la Figure 10 (où chaque point correspond à la valeur mesurée pour une expérience, le trait horizontal pour chaque série de valeurs représentant la moyenne arithmétique de la série considérée). Les dispersions sont tout à fait satisfaisantes, avec des valeurs moyennes (Emin et Emax, $\pm$ écart-type) de 1,0 $\pm$ 1,5% et 75,7 $\pm$ 6,0% pour le $^{51}$Cr et de 1,2 $\pm$ 1,2% et 73,6 $\pm$ 8,4% pour la nanoluciférase. Ces résultats montrent qu'Emin et Emax sont constants et reproductibles entre les essais, même lorsque des gammes de concentration 80% et 125% sont utilisées en plus de la gamme standard.

De la même façon, l'analyse de la dispersion des $EC_{50}$ des gammes 100% [Figure 11 : chaque point représente la valeur d'$EC_{50}$ obtenue dans une expérience utilisant soit la mesure de la nanoluciférase libérée (ronds noirs) soit celle du chrome-51 (carrés blancs), le trait horizontal représentant la moyenne arithmétique des 3 valeurs] montre des résultats très satisfaisants pour les deux méthodes et une valeur moyenne légèrement plus basse pour la méthode par luminescence : 55,2 $\pm$ 15,6 ng/mL pour le $^{51}$Cr et 31,8 $\pm$ 7,3 ng/mL pour la nanoluciférase (moyennes $\pm$ écarts-types), soit des coefficients de variation (CV) de 28,3% et de 22,8% respectivement.

Enfin, la potency est calculée pour chaque expérience indépendante comme le ratio (exprimé en pourcentages) entre l'$EC_{50}$ de l'échantillon de référence (ici la gamme standard 100%) et l'$EC_{50}$ de l'échantillon à tester (ici les gammes 80 et 125%). Les résultats sont représentés sur la Figure 12 : la potency calculée est reportée en ordonnées pour les deux méthodes, nanoluciférase (ronds noirs) et chrome-51 (carrés blancs), le trait horizontal (plein pour la méthode nanoluciférase et pointillé épais pour la méthode $^{51}$Cr) représente la moyenne arithmétique des 3 mesures. Les lignes pointillées fines indiquent les potency théoriques idéales de 80% et 125%).

Les résultats montrent là encore une dispersion comparable des mesures entre les deux méthodes et des résultats conformes aux attentes. En effet, les valeurs moyennes ($\pm$ écart-type) pour les gammes 80% et 125% sont de 76,7 $\pm$ 10% (CV = 14,2%) et 131,9 $\pm$ 11,9% (CV = 9,1%) pour la méthode $^{51}$Cr et de 83,6 $\pm$ 22% (CV = 26,4%) et 119,5 $\pm$ 22,7% (CV = 19,0%) pour la méthode nanoluciférase.

Ces résultats démontrent la pertinence de la méthode basée sur la libération de la nanoluciférase dans le modèle d'ADCC anti-CD20 décrit ici, dont les performances sont tout à fait comparables à celles de la méthode de référence au $^{51}$Cr.

**Exemple 5** :

En utilisant le même type de cellules cibles que celui de l'exemple 4, la méthode nanoluciférase a été comparée à la méthode au $^{51}$Cr dans un essai de CDC anti-CD20. La méthodologie appliquée a été la même que celle décrite dans l'exemple 4, le système cytolytique étant constitué par une source de complément. De la même façon, une gamme standard d'anticorps (rituximab) a été utilisée, ainsi que des gammes de concentrations modifiées à 80% et 125%. Trois expériences indépendantes ont été réalisées, dont l'une réalisée par un second opérateur.

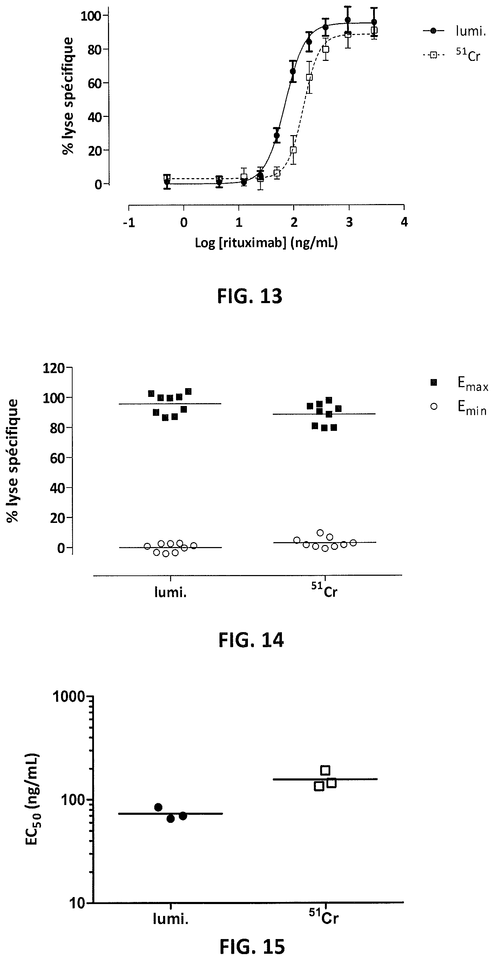

La Figure 13 montre la comparaison des courbes sigmoïdes obtenues à partir des valeurs moyennes issues des 3 expériences. Les écarts-types sont satisfaisants et les courbes sont tout à fait similaires dans leur aspect, malgré un léger décalage des $EC_{50}$.

L'analyse fine des $E_{min}$ et des $E_{max}$ (Figure 14) confirme la similarité de ces paramètres, avec 3,0 $\pm$ 3,4% et 88,6 $\pm$ 7,8% pour le $^{51}$Cr et -0,3 $\pm$ 3,2% et 95,6 $\pm$ 7,0% pour la nanoluciférase, respectivement (moyennes $\pm$ écart-type) pour l'ensemble des gammes 80, 100 et 125%.

L'analyse de la dispersion des $EC_{50}$ pour la gamme 100% dans les 3 expériences (Figure 15) donne elle aussi des résultats satisfaisants avec une valeur moyenne ($\pm$ écart-type) de 156,7 $\pm$ 30,1 ng/mL (CV = 19,2%) pour le $^{51}$Cr et de 73,1 $\pm$ 9,9 ng/mL (CV = 13,5%) pour la nanoluciférase.

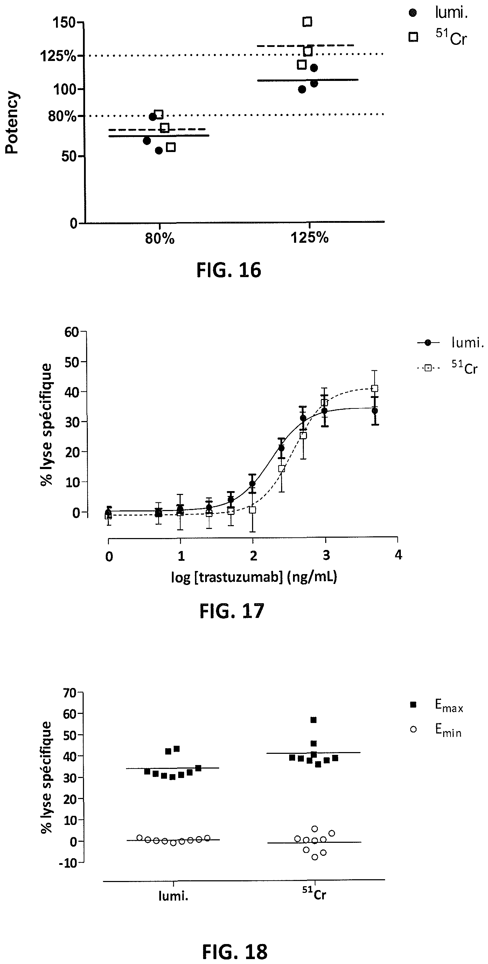

Enfin, le calcul des potencies (Figure 16) permet de constater des performances similaires entre les deux méthodes, avec des valeurs (moyennes $\pm$ écarts-types) tout à fait comparables pour la gamme 80% (69,6 $\pm$ 12,3% pour le $^{51}$Cr et 65,0 $\pm$ 12,9% pour la nanoluciférase, CV = 17,7% et 19,8% respectivement) et une meilleure précision avec la gamme 125% pour la méthode nanoluciférase en dépit d'une justesse un peu moins bonne (105,8 $\pm$ 8,2% [CV = 7,7%], contre 131,5 $\pm$ 16,4% [CV = 12,4%] pour le $^{51}$Cr).

Ces résultats démontrent la pertinence de la méthode basée sur la libération de la nanoluciférase dans le modèle de CDC anti-CD20 décrit ici, dont les performances sont tout à fait comparables à celles de la méthode de référence au $^{51}$Cr.

**Exemple 6** :

La méthode nanoluciférase a été comparée à la méthode au $^{51}$Cr dans un essai d'ADCC anti-HER2. Pour cela, des cellules SKOV3 exprimant stablement la nanoluciférase ont été marquées ou non au $^{51}$Cr et utilisées simultanément dans un essai d'ADCC dans les mêmes conditions expérimentales, selon le même principe que celui décrit dans l'exemple 4.

Des cellules cibles SKOV3 transformées pour exprimer stablement la nanoluciférase ont été incubées ou non 1 heure dans une solution saline de $Na_2CrO_4$ (Perkin Elmer, France), équivalant à 100 μCi de $^{51}$Cr par million de cellules, puis lavées 4 fois par du RPMI-1640, 10% SVF et comptées. Les cellules (3000 par puits), marquées ou non au $^{51}$Cr, ont ensuite été incubées à 37°C dans une atmosphère humidifiée à 5% de $CO_2$ en plaques 96 puits à fonds plats traitées pour la culture cellulaire, en présence de concentrations croissantes (1, 5, 10, 25, 50, 100, 250, 500, 1000 et 5000 ng/mL, dont les logarithme sont indiqués en abscisses) de l'anticorps trastuzumab (Herceptine®) et de cellules effectrices cytotoxiques (lymphocytes T exprimant CD16, 30000 cellules par puits) dans du RPMI-1640 supplémenté par 5% de SVF.

A l'issue de l'incubation de 4 heures, 25 μL de surnageant de chaque condition sont récupérés, la cytolyse est mesurée et le pourcentage de lyse spécifique calculé selon des modalités strictement identiques à celles décrites dans l'exemple 4.

Une gamme standard (100%) de trastuzumab ainsi que des variations à 80% et 125% de cette gamme ont été utilisées, et l'expérience a été répétée 3 fois de façon indépendante, dont l'une réalisée par un second opérateur. Les pourcentages de lyse spécifique moyens obtenus par les deux méthodes dans les trois expériences ainsi que les modélisations associées sont montrés sur la Figure 17. Là encore, les deux méthodes donnent des résultats très similaires, avec une dispersion des valeurs qui semble plus faible pour la méthode nanoluciférase.

Ces données sont confirmées par l'analyse des lyses minimales et maximales (Figure 18 : chaque point correspond à la valeur mesurée pour une expérience, le trait horizontal pour chaque série de valeurs représentant la moyenne arithmétique de la série considérée.), qui montre des valeurs (moyennes ± écarts-types) d'$E_{min}$ et d'$E_{max}$ de 0,5 ± 2,2% et 41,3 ± 4,3% pour le $^{51}$Cr et de 0,3 ± 0,7% et 34,1 ± 4,3% pour la nanoluciférase, respectivement.

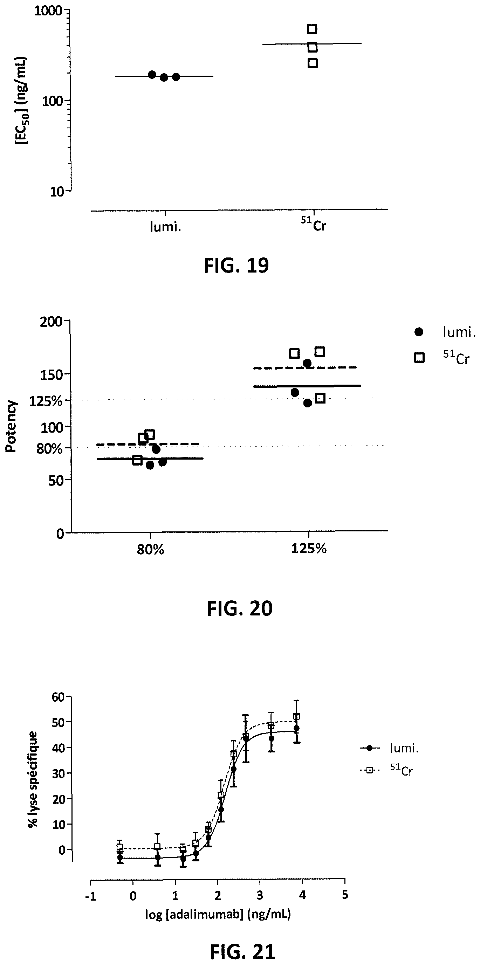

L'analyse des $EC_{50}$ (Figure 19: chaque point représente la valeur d'$EC_{50}$ obtenue dans une expérience), le trait horizontal représentant la moyenne arithmétique des 3 valeurs) confirme la plus faible dispersion obtenue dans ce modèle grâce à la méthode nanoluciférase. Ainsi, l'$EC_{50}$ moyen (± écart-type) pour la gamme standard est de 181,6 ± 8,0 ng/mL en nanoluciférase (CV = 4,4%) alors qu'il est de 402,5 ± 172,5 ng/mL pour le $^{51}$Cr, soit un CV de 42,8%.

Malgré cette dispersion plus élevée des $EC_{50}$ entre les expériences pour la méthode au chrome, l'analyse des potencies (qui se calculent expérience par expérience en divisant l'$EC_{50}$ de la gamme standard par celui de la gamme modifiée) ne montre pas de différence significative de performance entre les deux méthodes (Figure 20). Sur cette figure 20, le trait horizontal (plein pour la méthode nanoluciférase et pointillé épais pour la méthode $^{51}$Cr) représente la moyenne arithmétique des trois mesures. Les lignes pointillées fines indiquent les potency théoriques idéales de 80% et 125%. La potency moyenne (± écart-type) calculée pour les gammes 80% et 125% est de 82,9 ± 13,0% (CV = 15,7%) et 154,2 ± 24,8% (CV = 16,1%) pour la méthode au $^{51}$Cr et de 69,3 ± 7,9% (CV = 11,4%) et 136,9 ± 19,4% (CV = 14,2%) pour la méthode nanoluciférase, respectivement.

L'ensemble de ces résultats démontre l'utilité de la méthode nanoluciférase en remplacement de la méthode au $^{51}$Cr dans ce modèle d'ADCC anti-HER2.

**Exemple 7** :

La méthode de mesure de libération de la nanoluciférase a été comparée à la méthode au chrome dans un troisième modèle, celui de l'anticorps anti-TNF-alpha adalimumab (Humira®). Le présent exemple illustre sa mise en oeuvre dans un essai de mesure de l'activité CDC. Pour cela, des cellules CHO préalablement transformées et sélectionnées pour exprimer stablement le TNF-alpha humain sous sa forme membranaire ont été transformées pour exprimer stablement la nanoluciférase. La méthodologie suivie est ensuite comparable à celle décrite dans l'exemple 5. Brièvement, ces cellules, marquées ou non au $^{51}$Cr, ont été incubées en présence de concentrations croissantes d'adalimumab (Humira®) et d'une source de complément (de cobaye (Sigma)). La lyse spécifique a ensuite été mesurée par la méthode adéquate (libération du chrome ou de la nanoluciférase).

Le pourcentage de lyse spécifique obtenu pour chaque concentration X est calculé de la même façon pour les deux types de méthodes, à partir des mesures de radioactivité (en CCPM) ou de luminescence (en RLU) selon la formule

$$\frac{CCPM/RLU[X] - CCPM/RLU[spontané]}{CCPM/RLU[max] - CCPM/RLU[spontané]} \times 100.$$

Dans les deux cas, les conditions maximum et spontané ont été obtenues par incubation des cellules dans le même milieu contenant 8000 ng/mL de trastuzumab en remplacement de l'adalimumab (libération spontanée) et 0,01% de triton X-100 (dans le cas de la libération maximum uniquement). Les pourcentages de lyse spécifiques sont représentés en ordonnées, chaque point (rond noir pour la nanoluciférase et carrés blancs pour le $^{51}$Cr) représentant la moyenne des neuf déterminations (3 réplicats dans 3 expériences indépendantes) et les barres verticales représentant les écarts-types. Les données ont été modélisées à l'aide d'une régression sigmoidale à 4 paramètres en utilisant le logiciel GraphPad Prism.

Les résultats moyens obtenus pour les 3 expériences sont représentés sur la Figure 21. Ils montrent ici encore une très bonne comparabilité des deux méthodes, avec des courbes d'aspects et de caractéristiques très semblables (plateaux, pentes, $EC_{50}$).

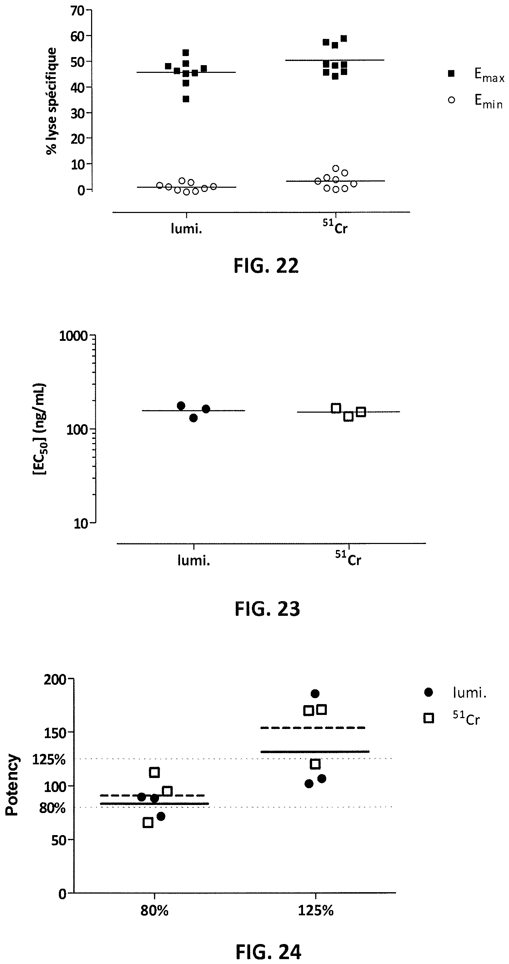

L'analyse de la dispersion des $E_{min}$ et $E_{max}$ réalisées confirme cette similarité (Figure 22) avec des valeurs moyennes (± écart-type, sur l'ensemble des gammes dans les 3 expériences) respectives de 3,2 ± 2,8% et 50,4 ± 5,6% pour le $^{51}$Cr et de 0,9 ± 1,5% et 45,7 ± 5,1% pour la nanoluciférase.

L'analyse de la dispersion des $EC_{50}$ des trois expériences de CDC décrites dans la Figure 21 (Figure 23) pour les gammes standard donne ici une égalité quasi-parfaite entre les deux méthodes, les valeurs moyennes (± écart-type, moyenne représentée par un trait horizontal) des 3 expériences étant de 150,8 ± 15 ng/mL pour le chrome (CV = 9,9%) et de 156,9 ± 23,4 ng/mL pour la nanoluciférase (CV = 14,9%).

Enfin, l'analyse des potencies à travers les gammes 80% et 125% montre elle aussi des performances comparables entre les deux méthodes (Figure 24) avec des potencies moyennes (± écart-type) de 91,2 ± 23,6% (CV = 25,8%) et 153,9 ± 29.2% (CV = 19,0%) pour le $^{51}$Cr et de 83,4 ± 10,1% (CV = 12,1%) et 131,7 ± 47,2% (CV = 35,8%) pour la nanoluciférase (respectivement pour les gammes 80% et 125%).

Ces résultats démontrent que la méthode basée sur la mesure de la libération de nanoluciférase est équivalente en termes de résultats et de performances à la méthode de libération du $^{51}$Cr pour la mesure de l'activité CDC dans un modèle anti-TNF-alpha.

**Exemple 8** :

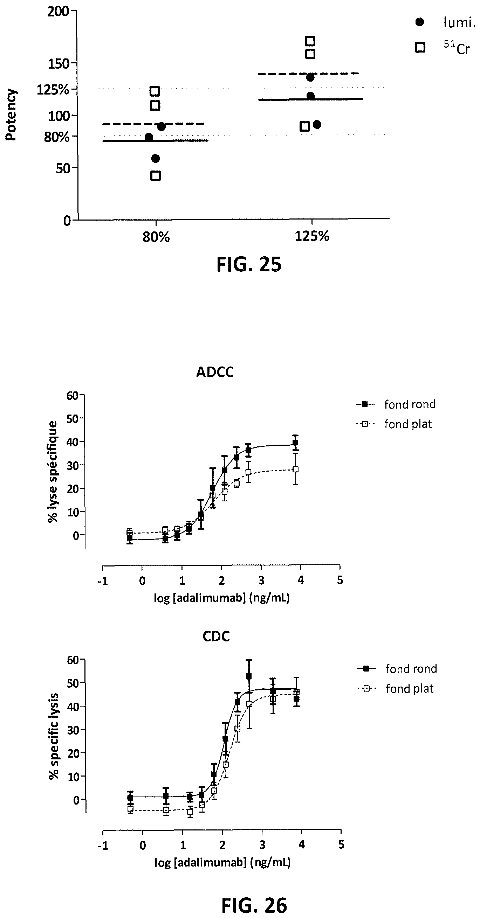

La méthode nanoluciférase a été comparée à la méthode au $^{51}$Cr dans un essai d'ADCC anti-TNF-alpha. Pour cela, les cellules CHO décrites dans l'exemple 7, exprimant stablement le TNF-alpha et la nanoluciférase, ont été utilisées, ainsi qu'une gamme standard d'adalimumab et des variations à 80% et 125% de cette gamme. Trois expériences indépendantes ont été réalisées, en suivant la même méthodologie que dans les exemples précédents. A partir des $EC_{50}$ générés dans les modèles de régression sigmoïdale à partir des données de lyse spécifique, les potencies des gammes 80% et 125% ont été calculées comme précédemment et les résultats sont présentés dans la Figure 25.

Les résultats sont comparables entre les 2 méthodes, avec un léger avantage en termes de dispersion des valeurs pour la méthode nanoluciférase. En effet, les potencies moyennes (± écarts-types) des gammes 80% et 125% calculées sont de 91,4 ± 43,3% (CV = 47,4%) et 138,5 ± 43,9% (CV = 31,7%) pour la méthode $^{51}$Cr et de 75,5 ± 15,5% (CV = 20,6%) et 114,2 ± 22,8% (CV = 19,9%) pour la nanoluciférase. Ces résultats démontrent l'utilité de la méthode nanoluciférase pour mesurer l'activité ADCC des anticorps anti-TNF-alpha en remplacement de la méthode au [51]Cr.

**Exemple 9** :

Cet exemple illustre l'adéquation entre la méthode nanoluciférase et les phénomènes biologiques mis en oeuvre dans les mécanismes d'ADCC et de CDC. Pour cela, des réactions d'ADCC et de CDC ont été réalisées en parallèle et dans les mêmes conditions expérimentales en utilisant pour l'incubation soit des plaques à fond plat soit des plaques à fond rond, ces dernières favorisant les contacts et les interactions cellulaires grâce à la forme particulière du puits. Les résultats sont présentés sur la Figure 26.

Ils montrent que, dans le cas de l'essai d'ADCC pour lequel les interactions entre cellules effectrices et cibles sont indispensables à l'effet cytotoxique, la forme du puits influence effectivement le pourcentage de lyse maximal (38,1% en moyenne pour les fonds ronds contre 27,6% pour les fonds plats), sans que ce phénomène ne modifie significativement les $EC_{50}$ résultantes (59,8 ng/mL contre 65,3 ng/mL, respectivement). Au contraire, la forme du puits n'influence pas la réaction de CDC, avec des $E_{max}$ moyens (47,0% en fonds ronds et 44,6% en fonds plats) et des $EC_{50}$ moyens (114,1 et 155,3 ng/mL, respectivement) similaires et indépendants de la forme du puits.

Favoriser les contacts entre les cellules par une forme de puits adaptée permet donc bien d'améliorer la quantité de cellules cibles lysées par ADCC et détectées par la méthode nanoluciférase. De façon cohérente, l'essai de CDC, dans lequel la seule population cellulaire présente est la population de cellules cibles et dans lequel le système effecteur est soluble (le complément), est quant à lui insensible à la forme du puits.

**Exemple 10** :

Afin de comparer les performances de la méthode de libération de la nanoluciférase à celles de la méthode de référence par libération de $^{51}$Cr, les résultats de variabilité présentés dans les exemples 4 à 8 ont été rassemblés dans le Tableau 2 ci-après.

**Tableau 2**

| Modèle | Essai | CV sur EC50 | CV sur Potency 80% (et biais relatif) | CV sur Potency 125% (et biais relatif) |
|---|---|---|---|---|
| | | $^{51}$Cr | | |
| CD20 | ADCC | 28,3% | 14,2% (-4,1%) | 26,4% (+4,5%) |
| | CDC | 19,2% | 17,7% (-13,0%) | 12,4% (+5,2%) |
| HER2 | ADCC | 42,8% | 15,7% (+3,6%) | 16,1% (+23,4%) |
| TNF-alpha | ADCC | 19,9% | 47,4% (+14,3%) | 31,7% (+10,8%) |
| | CDC | 9,9% | 25,8% (+14,0%) | 19,0% (+23,1%) |
| | | nanoluciférase | | |
| Modèle | Essai | CV sur EC50 | CV sur Potency 80% (et biais relatif) | CV sur Potency 125% (et biais relatif) |
| CD20 | ADCC | 22,8% | 9,1% (+5,5%) | 19,0% (-4,4%) |
| | CDC | 13,5% | 19,8% (-18,8%) | 7,7% (-15,4%) |
| HER2 | ADCC | 4,4% | 11,4% (-13,4%) | 14,2% (+9,5%) |
| TNF-alpha | ADCC | 14,5% | 20,6% (-5,6%) | 19,9% (-8,6%) |
| | CDC | 14,9% | 12,1% (+4,3%) | 35,8% (+5,4%) |

Tous les résultats sont exprimés en pourcentages. Trois expériences indépendantes ont été réalisées dans chacun des modèles et essais. Les coefficients de variation (CV) des $EC_{50}$ et potencies sont calculés comme l'écart-type divisé par la moyenne des données correspondantes. Le biais relatif exprime l'écart entre la valeur attendue et celle réellement obtenue, en pourcentage de la valeur attendue, selon la formule suivante (où P représente la potency) :

$$Biais\ relatif = \frac{P\ moyen - P\ théorique}{P\ théorique} \times 100$$

Le but des exemples 11 et 12 ci-après est d'évaluer la capacité de la présente méthode à mesurer des phénomènes de mort cellulaire à cinétiques plus longues que celles mises en jeu dans les mécanismes d'ADCC ou de CDC, par exemple dans le cas de la détection de l'effet cytotoxique de molécules polluantes, toxiques ou pro-apoptotiques.

<u>**Exemple 11**</u> : agent physique

Des cellules Raji transformées pour exprimer stablement la nanoluciférase (50000 cellules par puits) sont cultivées 48 heures à 37°C (sous atmosphère humidifiée contenant 5% de CO2) en milieu RPMI-1640 contenant 10% de SVF, et en présence ou non de 0,2% de triton X-100 (pour déterminer le relargage maximal), après avoir été exposées à un rayonnement ultra-violet (UV) pendant un temps variable (0, 10, 20, 30, 40, 50 ou 60 secondes). Après 48 h, 25 $\mu$L de surnageant sont récupérés dans chaque puits, transférés dans une plaque "demi-puits" blanche, mélangés à 25 $\mu$L de substrat NanoGlo® dilué au 1/50 en D-PBS, incubés 3 minutes et lus sur un luminomètre Mithras LB940 (Berthold Technologies). Les résultats de la lecture sont exprimés en RLU ("relative luminescence unit"). Le pourcentage de cellules mortes pour chaque concentration de staurosporine est déterminé selon la formule (RLU[milieu sans triton])/(RLU[0,2% triton]) x100.

Après prélèvement du surnageant, le reste des cellules est remis en suspension, mélangé extemporanément avec 0,25 $\mu$M final du marqueur vital TO-PRO-3 (Life Technologies) et acquis sur un cytomètre en flux C6 (BD/Accuri). Le pourcentage de cellules mortes est déterminé lors de l'analyse des données de cytométrie comme le pourcentage de cellules ayant intégré le TO-PRO-3.

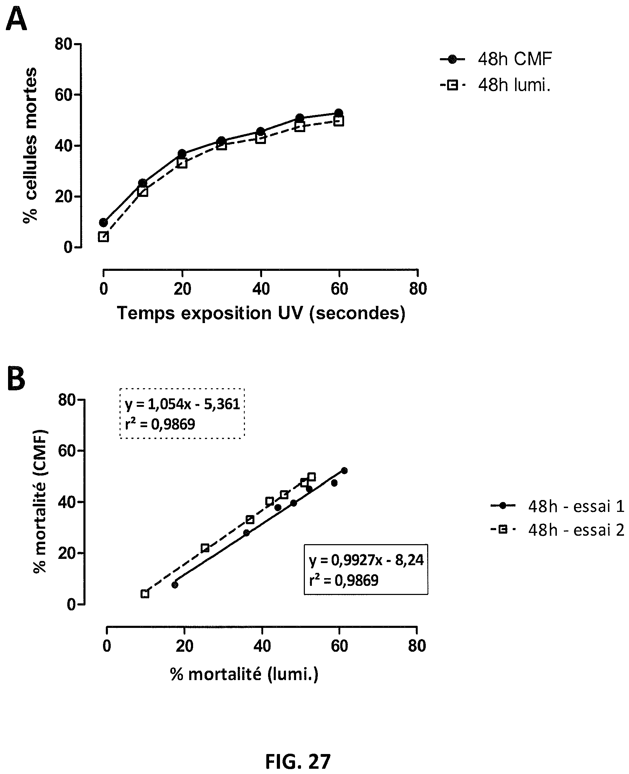

Deux expériences identiques indépendantes ont été réalisées, dont les résultats sont présentés sur les graphes A et B de la figure 27. Le graphe du haut (A) représente les pourcentages de cellules mortes déterminés selon les 2 méthodes (luminescence de la nanoluciférase ["lumi.", trait pointillé] ou cytométrie en flux ["CMF", trait plein]) en fonction du temps d'exposition aux UV pour un essai représentatif. Le graphe du bas (B) représente le pourcentage de cellules mortes déterminé par la méthode luminescente ("% mortalité lumi.") en fonction du pourcentage correspondant de cellules mortes mesuré par cytométrie en flux ("% mortalité CMF") pour l'ensemble des conditions dans l'essai 1 (trait plein, ronds pleins) et dans l'essai 2 (trait pointillé, carrés vides). Une régression linéaire a été calculée entre ces deux paramètres grâce au logiciel GraphPad Prism et tracée sur le graphe (l'équation de la droite et le coefficient de régression linéaire $R^2$ sont représentés sur le graphe pour chacun des 2 essais).

**Exemple 12** : agent chimique

Des cellules Raji transformées pour exprimer stablement la nanoluciférase (50000 cellules par puits) sont cultivées 48 heures à 37°C (sous atmosphère humidifiée contenant 5% de CO2) en présence de concentrations croissantes de staurosporine, une drogue pro-apoptotique, en milieu RPMI-1640 contenant 10% de SVF, et en présence ou non de 0,2% de triton X-100 (pour déterminer le relargage maximal). A la fin de l'incubation, 25 µL de surnageant sont récupérés dans chaque puits, transférés dans une plaque "demi-puits" blanche, mélangés à 25 µL de substrat NanoGlo® dilué au 1/50 en D-PBS, incubés 3 minutes et lus sur un luminomètre Mithras LB940. Les résultats de la lecture sont exprimés en RLU ("relative luminescence unit"). Le pourcentage de cellules mortes pour chaque concentration de staurosporine est déterminé selon la formule (RLU[milieu sans triton])/(RLU[0,2% triton]) $\times$ 100.

Après prélèvement du surnageant, le reste des cellules est remis en suspension, mélangé extemporanément avec 0,25 µM final du marqueur vital TO-PRO-3 (Life Technologies) et acquis sur un cytomètre en flux C6 (BD/Accuri). Le pourcentage de cellules mortes est déterminé lors de l'analyse des données de cytométrie comme le pourcentage de cellules ayant intégré le TO-PRO-3.

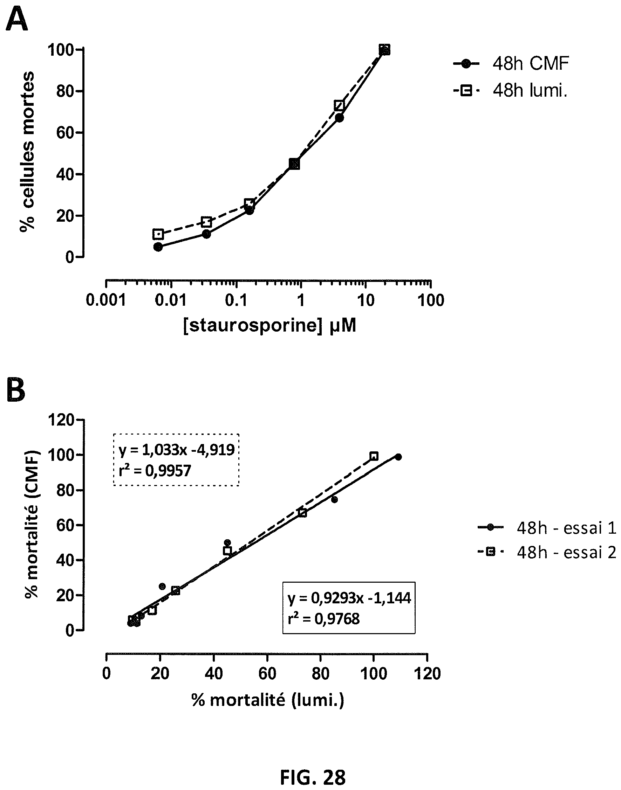

Deux expériences identiques indépendantes ont été réalisées, dont les résultats sont présentées sur les graphes A et B de la figure 28. Le graphe du haut (A) représente les pourcentages de cellules mortes déterminés selon les 2 méthodes (luminescence de la nanoluciférase ["lumi.", trait pointillé] ou cytométrie en flux ["CMF", trait plein]) en fonction de la concentration en staurosporine en présence de laquelle les cellules ont été incubées, pour un essai représentatif. Le graphe du bas (B) représente le pourcentage de cellules mortes déterminé par la méthode luminescente ("% mortalité lumi.") en fonction du pourcentage correspondant de cellules mortes mesuré par cytométrie en flux ("% mortalité CMF") pour l'ensemble des conditions dans l'essai 1 (trait plein, ronds pleins) et dans l'essai 2 (trait pointillé, carrés vides). Une régression linéaire a été calculée entre ces deux paramètres grâce au logiciel GraphPad Prism et tracée sur le graphe (l'équation de la droite et le coefficient de régression linéaire R2 sont représentés sur le graphe pour chacun des 2 essais).

Ces résultats montrent d'une part qu'il existe bien une relation proportionnelle entre la concentration de staurosporine et la mortalité des cellules cibles, quelle que soit la méthode de mesure de la mortalité. D'autre part, la mortalité détectée par la mesure de la nanoluciférase est parfaitement corrélée à l'analyse au niveau cellulaire par cytométrie en flux. En effet, la pente de la droite de régression est proche de 1 (pente = 0,86), de même que le coefficient de régression linéaire ($R^2$ = 0,9659).

Des résultats similaires (non représentés) ont été obtenus avec les lignées cibles SKOV3 et CHO-TNF-alpha exprimant la nanoluciférase.

**Revendications**

1. Procédé non radioactif de détermination (contrôle et quantification) in vitro directe de l'action cytolytique d'un agent actif vis-à-vis de cellules cibles et/ou d'un milieu environnant des cellules cibles, comprenant les étapes successives ci-après :

   i) Transformation génétique de cellules cibles pour exprimer une enzyme exogène aux dites cellules cibles,
   ii) Exposition des dites cellules cibles génétiquement transformées à l'agent actif et/ou au dit milieu environnant à tester, pouvant conduire à la lyse d'au moins une partie des cellules cibles en libérant ladite enzyme exogène dans le milieu extracellulaire,
   iii) Mesure de l'activité de l'enzyme exogène libérée lors de la lyse desdites cellules cibles

   **caractérisé en ce que** ladite enzyme exogène est une enzyme de masse molaire inférieure ou égale à 45 kDa possédant une séquence peptidique présentant au moins 60 % d'homologie avec la séquence peptidique sauvage de la sous-unité 19 kDa de la luciférase produite par la crevette *Oplophorus gracilirostris*, et dont l'activité est détectable par luminescence ou fluorescence et **en ce que** ladite enzyme exogène est exprimée seule et sous forme libre dans le cytoplasme desdites cellules cibles.

2. Procédé selon la revendication 1, **caractérisé en ce que** la masse molaire de l'enzyme exogène est inférieure ou égale à 30 kDa, de préférence inférieure ou égale à 25 kDa, de préférence encore inférieure ou égale à 20 kDa.

3. Procédé selon l'une quelconque des revendications 1 ou 2, **caractérisé en ce que** ladite enzyme exogène est une luciférase.

4. Procédé selon l'une quelconque des revendications 1 à 3, **caractérisé en ce que** ladite enzyme exogène possède une séquence peptidique présentant au moins 80 % d'homologie, avec la séquence peptidique sauvage de la sous-unité 19 kDa de la luciférase produite par la crevette *Oplophorus gracilirostris.*

5. Procédé selon la revendication 4, **caractérisé en ce que** ladite enzyme exogène est sous une forme à activité et stabilité optimisée, commercialisée par la société Promega sous le nom NanoLuc®.

6. Procédé selon l'une quelconque des revendications précédentes, **caractérisé en ce que** la quantité d'enzyme exogène libérée est mesurée par clivage d'un substrat spécifique de ladite enzyme, le substrat étant de préférence de la famille de la coelentérazine, plus particulièrement de la famille de la furimazine et de ses dérivés.

7. Procédé selon l'une quelconque des revendications précédentes, **caractérisé en ce que** le milieu à tester environnant les cellules cibles comprend un agent biologique et/ou un agent chimique et/ou un agent physique potentiellement actif(s) vis-à-vis des dites cellules cibles.

8. Procédé selon la revendication 7, **caractérisé en ce que** l'agent biologique est un anticorps, de préférence choisi parmi les anticorps à visée thérapeutique, notamment les anticorps monoclonaux à visée thérapeutique.

9. Procédé selon la revendication 8, **caractérisé en ce que** les anticorps sont des anticorps dirigés contre des molécules exprimées principalement par les lymphocytes B normaux ou pathologiques, telles que CD19, CD20 ou IL-6R (CD126), de préférence CD20.

10. Procédé selon la revendication 8, **caractérisé en ce que** les anticorps sont des anticorps dirigés contre des molécules exprimées principalement par les lymphocytes T normaux ou pathologiques, telles que CD3, CD25 ou LPAM ("Lymphocyte Peyer Patch Adhésion Molecule" ou intégrine alpha[4]beta[7]).

11. Procédé selon la revendication 8, **caractérisé en ce que** les anticorps sont des anticorps dirigés contre des molécules exprimées principalement par des cellules d'origine lymphoïde normales ou pathologiques, telles que CTLA-4 (CD152), PD-1 (CD279) ou CD30 humaines

12. Procédé selon la revendication 8, **caractérisé en ce que** les anticorps sont des anticorps dirigés contre des molécules exprimées principalement par des cellules d'origine lymphoïde et myéloïde normales ou pathologiques, telles que CD52, VLA4 (CD49d) ou LFA-1 (CD11a) humaines.

13. Procédé selon la revendication 8, **caractérisé en ce que** les anticorps sont des anticorps dirigés contre des molécules exprimées principalement par des cellules carcinoïdes, telles que -EGFR ("Epidermal Growth Factor Receptor", aussi noté HER1 ou ErbB1), EGFR2 (ou HER2, ou ErbB2), EGFR-3 (ou HER3, ou ErbB3) ou EpCAM (CD326).

14. Procédé selon la revendication 8, **caractérisé en ce que** l'anticorps est dirigé contre la molécule TNF-alpha.

15. Procédé selon la revendication 8, **caractérisé en ce que** l'anticorps est dirigé contre la molécule VEGF ("Vascular Endothelial Growth Factor") ou son récepteur VEGFR.

16. Procédé selon la revendication 7, **caractérisé en ce que** l'agent chimique et/ou physique est choisi parmi les agents de chimiothérapie ou les molécules anti-cancéreuses, de préférence des molécules cytotoxiques ou des molécules de la famille des inhibiteurs des protéines kinases.

17. Procédé selon l'une quelconque des revendications précédentes dans lequel les cellules cibles sont transformées génétiquement afin d'exprimer transitoirement ou constitutivement ladite enzyme exogène sous une forme cytoplasmique et non sécrétée.

18. Procédé selon la revendication 17, **caractérisé en ce que** l'étape de transformation génétique desdites cellules cibles comprend l'introduction dans les dites cellules d'un vecteur d'expression portant la séquence codante de l'enzyme exogène et un promoteur de type constitutif permettant sa transcription dans une cellule, telle qu'une cellule eucaryote.

**19.** Procédé selon la revendication 18, **caractérisé en ce que** le vecteur est un vecteur viral, ou un vecteur plasmidique, de préférence un vecteur plasmidique.

**20.** Procédé selon l'une des revendications 18 ou 19, **caractérisé en ce que** le vecteur d'expression de l'enzyme exogène comprend un gène de résistance aux antibiotiques.

**21.** Procédé selon la revendication 20, **caractérisé en ce que** le gène de résistance aux antibiotiques est un gène de résistance à la généticine (G418), la puromycine, la blasticidine, l'hygromycine B, l'acide mycophénolique ou la zéocine, de préférence un gène de résistance à la puromycine.

**22.** Procédé selon l'une quelconque des revendications 19 à 21, **caractérisé en ce que** l'introduction dudit vecteur d'expression dans lesdites cellules est réalisée par infection par des particules virales portant le gène de l'enzyme exogène lorsque le vecteur d'expression est un vecteur viral, ou par des méthodes chimiques ou physiques lorsque le vecteur d'expression est un vecteur plasmidique.

**23.** Procédé selon la revendication 22, **caractérisé en ce que**, lorsque le vecteur est un vecteur plasmidique, l'introduction dans les cellules cibles est réalisée par électroporation.

**24.** Utilisation du procédé selon l'une quelconque des revendications précédentes pour mesurer la cytotoxicité cellulaire dépendante d'anticorps (ADCC), mesurer la cytotoxicité dépendante du complément (CDC) et/ou mesurer l'apoptose.

**25.** Kit pour la réalisation du procédé selon l'une quelconque des revendications 1 à 16 ou son utilisation selon la revendication 24, **caractérisé en ce qu'**il comporte :

- une lignée de cellules cibles exprimant l'enzyme exogène,
- des cellules effectrices cytotoxiques pour la réalisation d'un test d'ADCC et/ou une source de complément pour la réalisation d'un essai de CDC,
- un substrat activant ladite enzyme exogène pour produire une émission de lumière, et les éventuelles solutions tampons associées,
- une notice explicative.

**Patentansprüche**

**1.** Nichtradioaktives Verfahren zur direkten in vitro-Bestimmung (Kontrolle und Quantifizierung) der zytolytischen Wirkung eines aktiven Mittels gegenüber Zielzellen und/oder einem umgebenden Milieu der Zielzellen, umfassend die folgenden aufeinanderfolgenden Schritte:

i) genetische Transformation von Zielzellen, um ein gegenüber den Zielzellen exogenes Enzym zu exprimieren,
ii) Exposition der genetisch transformierten Zielzellen gegenüber dem zu testenden aktiven Mittel und/oder umgebenden Milieu, was zu der Lyse von mindestens einem Teil der Zielzellen durch Freisetzen des exogenen Enzyms in das extrazelluläre Milieu führen kann,
iii) Messung der Aktivität des bei der Lyse der Zielzellen freigesetzten exogenen Enzyms,

**dadurch gekennzeichnet, dass** das exogene Enzym ein Enzym mit einer molaren Masse von unter oder gleich 45 kDa ist mit einer Peptidsequenz, aufweisend mindestens 60 % Homologie mit der wilden Peptidsequenz der Untereinheit 19 kDa der Luciferase, produziert von der Garnele *Oplophorus gracilirostris,* und dessen Aktivität durch Lumineszenz oder Fluoreszenz detektierbar ist und dass das exogene Enzym allein und in freier Form im Zytoplasma der Zielzellen exprimiert ist.

**2.** Verfahren nach Anspruch 1, **dadurch gekennzeichnet, dass** die molare Masse des exogenen Enzyms kleiner oder gleich 30 kDa, vorzugsweise kleiner oder gleich 25 kDa, noch vorzugsweiser kleiner oder gleich 20 kDa ist.

**3.** Verfahren nach einem der Ansprüche 1 oder 2, **dadurch gekennzeichnet, dass** das exogene Enzym eine Luciferase ist.

**4.** Verfahren nach einem der Ansprüche 1 bis 3, **dadurch gekennzeichnet, dass** das exogene Enzym eine Peptidsequenz besitzt, die mindestens 80 % Homologie, mit der wilden Peptidsequenz der Untereinheit 19 kDa der von der Garnele *Oplophorus gracilirostris* produzierten Luciferase aufweist.

**5.** Verfahren nach Anspruch 4, **dadurch gekennzeichnet, dass** das exogene Enzym in einer Form mit Aktivität und optimierter Stabilität ist, vertrieben von der Firma Promega unter der Bezeichnung NanoLuc®.

**6.** Verfahren nach einem der vorangehenden Ansprüche, **dadurch gekennzeichnet, dass** die Menge freigesetzten exogenen Enzyms durch Spalten eine spezifischen Substrats des Enzyms gemessen wird, wobei das Substrat vorzugsweise aus der Familie des Coelenterazins, genauer aus der Familie des Furimazins und seiner Derivate ist.

**7.** Verfahren nach einem der vorangehenden Ansprüche, **dadurch gekennzeichnet, dass** das zu testende Milieu, welches die Zielzellen umgibt, ein biologisches Mittel und/oder ein chemisches Mittel und/oder ein physikalisches Mittel umfasst, das/die potentiell gegenüber den Zielzellen aktiv ist/sind.

**8.** Verfahren nach Anspruch 7, **dadurch gekennzeichnet, dass** das biologische Mittel ein Antikörper, vorzugsweise ausgewählt aus den Antikörpern mit therapeutischer Zielstellung, vor allen den monoklonalen Antikörpern mit therapeutischer Zielstellung, ist.

**9.** Verfahren nach Anspruch 8, **dadurch gekennzeichnet, dass** die Antikörper Antikörper sind, die gegen Moleküle gerichtet sind, die hauptsächlich von den normalen oder pathologischen Lymphozyten B wie CD19, CD20 oder IL-6 (CD126), vorzugsweise CD20, exprimiert werden.

**10.** Verfahren nach Anspruch 8, **dadurch gekennzeichnet, dass** die Antikörper Antikörper sind, die gegen Moleküle gerichtet sind, die hauptsächlich von den normalen oder pathologischen Lymphozyten T wie CD3, CD25 oder LPAM ("Lymphocyte Peyer Patch Adhesion Molecule" oder Integrin alpha[4] beta[7]) exprimiert werden.

**11.** Verfahren nach Anspruch 8, **dadurch gekennzeichnet, dass** die Antikörper Antikörper sind, die gegen Moleküle gerichtet sind, die hauptsächlich von normalen oder pathologischen humanen Zellen lymphoiden Ursprungs wie CTLA-4 (CD152), PD-1 (CD279) oder CD30 exprimiert werden.

**12.** Verfahren nach Anspruch 8, **dadurch gekennzeichnet, dass** die Antikörper Antikörper sind, die gegen Moleküle gerichtet sind, die hauptsächlich von normalen oder pathologischen humanen Zellen lymphoiden und myeloiden Ursprungs wie CD52, VLA4 (CD49d) oder LFA-1 (CD11a) exprimiert werden.

**13.** Verfahren nach Anspruch 8, **dadurch gekennzeichnet, dass** die Antikörper Antikörper sind, die gegen Moleküle gerichtet sind, die hauptsächlich von carcinoiden Zellen wie -EGFR ("Epidermal Growth Factor Receptor", auch HER1 oder ErbB1), EGFR2 (oder HER2, oder ErbB2), EGFR-3 (oder HER3, oder ErbB3) oder EpCAM (CD326) exprimiert werden.

**14.** Verfahren nach Anspruch 8, **dadurch gekennzeichnet, dass** der Antikörper gegen das Molekül TNF-alpha gerichtet ist.

**15.** Verfahren nach Anspruch 8, **dadurch gekennzeichnet, dass** der Antikörper gegen das Molekül VEGF ("Vascular Endothelial Growth Factor") oder seinen Rezeptor VEGFR gerichtet ist.

**16.** Verfahren nach Anspruch 7, **dadurch gekennzeichnet, dass** das chemische und/oder physikalische Mittel aus den Chemotherapie-Mitteln oder den Anti-Krebs-Molekülen, vorzugsweise cytotoxischen Molekülen oder Molekülen aus der Familie der Kinaseproteininhibitoren, ausgewählt ist.

**17.** Verfahren nach einem der vorangehenden Ansprüche, wobei die Zielzellen genetisch transformiert werden, um transitorisch oder konstitutiv das exogene Enzym in einer cytoplasmischen und nicht sekretierten Form zu exprimieren.

**18.** Verfahren nach Anspruch 17, **dadurch gekennzeichnet, dass** der Schritt der genetischen Transformation der Zielzellen die Einführung in die Zellen eines Expressionsvektors umfasst, welcher die codierende Sequenz des exogenen Enzyms und einen Promoter vom Typ konstitutiv umfasst, welche seine Transkription in eine Zelle wie eine eukaryotische Zelle erlauben.

**19.** Verfahren nach Anspruch 18, **dadurch gekennzeichnet, dass** der Vektor ein viraler Vektor oder ein plasmidischer Vektor, vorzugsweise ein plasmidischer Vektor, ist.

**20.** Verfahren nach einem der Ansprüche 18 oder 19, **dadurch gekennzeichnet, dass** der Expressionsvektor des exogenen Enzyms ein antibiotikaresistentes Gen umfasst.

**21.** Verfahren nach Anspruch 20, **dadurch gekennzeichnet, dass** das antibiotikaresistente Gen ein Resistenzgen gegenüber Geneticin (G418), Puromycin, Blasticidin, Hygromycin B, Mycophenolsäure oder Zeocin, vorzugsweise ein Resistenzgen gegenüber Puromycin, ist.

**22.** Verfahren nach einem der Ansprüche 19 bis 21, **dadurch gekennzeichnet, dass** die Einführung des Expressionsvektors in die Zellen durch Infektion durch virale Partikel, welche das Gen des exogenen Enzyms tragen, durchgeführt wird, wenn der Expressionsvektor ein viraler Vektor ist, oder durch chemische oder physikalische Methoden, wenn der Expressionsvektor ein plasmidischer Vektor ist.

**23.** Verfahren nach Anspruch 22, **dadurch gekennzeichnet, dass**, wenn der Vektor ein plasmidischer Vektor ist, die Einführung in die Zielzellen durch Elektroporation durchgeführt wird.

**24.** Verwendung des Verfahrens nach einem der vorangehenden Ansprüche zum Messen der zellulären Zytotoxizität in Abhängigkeit vom Antikörper (ADCC), Messen der Zytotoxizität in Abhängigkeit vom Komplement (CDC) und/oder Messen der Apoptose.

**25.** Kit für die Durchführung des Verfahrens nach einem der Ansprüche 1 bis 16 oder seine Verwendung nach Anspruch 24, **dadurch gekennzeichnet, dass** es aufweist:

- eine Linie von Zielzellen, die das exogene Enzym exprimieren,
- Zellen mit zytotoxischem Effekt für die Durchführung eines ADCC-Tests und/oder eine Komplementquelle für die Durchführung eines CDC-Versuchs,
- ein Substrat, welches das exogene Enzym aktiviert, um eine Lichtemission hervorzurufen, und die eventuell zugeordneten Pufferlösungen,
- eine Anleitung.

**Claims**

**1.** Non-radioactive method for direct *in vitro* determination (control and quantification) of the cytolytic action of an agent active against target cells and/or a medium surrounding target cells, comprising the following successive steps:

i) Genetically transforming target cells to express an enzyme exogeneous to said target cells;
ii) Exposing said genetically transformed target cells to the active agent and/or to said surrounding medium to be assayed, which can result in the lysis of at least part of the target cells while releasing said exogeneous enzyme in the extracellular medium;
iii) Measuring the activity of the exogenous enzyme released on lysis of said target cells;

**characterized in that** said exogenous enzyme is an enzyme of molar mass equal to or lower than 45 kDa with a peptide sequence having at least 60 % homology with the wild-type peptide sequence of the 19 kDa sub-unit of luciferase produced by the shrimp *Oplophorus gracilirostris,* the activity of which can be detected by luminescence or fluorescence, and **in that** said exogenous enzyme is expressed alone and in free form in the cytoplasm of said target cells.

**2.** The method as claimed in claim 1, **characterized in that** the molar mass of the exogenous enzyme is less than or equal to 30 kDa, preferably less than or equal to 25 kDa, more preferably less than or equal to 20 kDa.

**3.** The method as claimed in either one of claims 1 and 2, **characterized in that** said exogenous enzyme is a luciferase.

**4.** The method as claimed in any one of claims 1 to 3, **characterized in that** said exogenous enzyme has a peptide sequence which exhibits at least 80% homology, with the wild-type peptide sequence of the 19 kDa subunit of the luciferase produced by the shrimp *Oplophorus gracilirostris.*

5. The method as claimed in claim 4, **characterized in that** said exogenous enzyme is in a form with optimized activity and stability, sold by the company Promega under the name NanoLuc®.

6. The method as claimed in any one of the preceding claims, **characterized in that** the amount of exogenous enzyme released is measured by cleavage of a substrate specific for said enzyme, the substrate preferably being of the coelenterazine family, more particularly of the family of 25 furimazine and derivatives thereof.

7. The method as claimed in any one of the preceding claims, **characterized in that** the medium to be tested that surrounds the target cells comprises a biological agent and/or a chemical agent and/or a physical agent which is (are) potentially active with respect to said target cells.

8. The method as claimed in claim 7, **characterized in that** the biological agent is an antibody, preferably chosen from antibodies for therapeutic purposes, in particular monoclonal 5 antibodies for therapeutic purposes.

9. The method as claimed in claim 8, **characterized in that** the antibodies are antibodies directed against molecules expressed mainly by normal or pathological B lymphocytes, such as CD19, CD20 or IL-6R (CD126), preferably CD20.

10. The method as claimed in claim 8, **characterized in that** the antibodies are antibodies 10 directed against molecules expressed mainly by normal or pathological T lymphocytes, such as CD3, CD25 or LPAM (Lymphocyte Peyer's Patch Adhesion Molecule or alpha[4]beta[7]-integrin).

11. The method as claimed in claim 8, **characterized in that** the antibodies are antibodies directed against molecules expressed mainly by normal or pathological cells of lymphoid 15 origin, such as human CTLA-4 (CD152), PD-1 (CD279) or CD30.

12. The method as claimed in claim 8, **characterized in that** the antibodies are antibodies directed against molecules mainly expressed by normal or pathological cells of lymphoid and myeloid origin, such as human CD52, VLA4 (CD49d) or LFA-1 (CD11a).

13. The method as claimed in claim 8, **characterized in that** the antibodies are antibodies 20 directed against molecules expressed mainly by carcinoid cells, such as EGFR (Epidermal Growth Factor Receptor, also denoted HER1 or ErbB1), EGFR2 (or HER2, or ErbB2), EGFR-3 (or HER3, or ErbB3) or EpCAM (CD326).

14. The method as claimed in claim 8, **characterized in that** the antibody is directed against the TNF-alpha molecule.

15. The method as claimed in claim 8, **characterized in that** the antibody is directed against the VEGF (Vascular Endothelial Growth Factor) molecule or its VEGFR receptor.

16. The method as claimed in claim 7, **characterized in that** the chemical and/or physical agent is chosen from chemotherapy agents or anti-cancer molecules, preferably cytotoxic molecules or molecules of the protein kinase inhibitor family.

17. The method as claimed in any one of the preceding claims, wherein the target cells are genetically transformed in order to transiently or constitutively express said exogenous 5 enzyme in a cytoplasmic and non-secreted form.

18. The method as claimed in claim 17, **characterized in that** the step of genetically transforming said target cells comprises introducing into said cells an expression vector carrying the coding sequence of the exogenous enzyme and a promoter of constitutive type which allows its transcription in a cell, such as a eukaryotic cell.

19. The method as claimed in claim 18, **characterized in that** the vector is a viral vector or a plasmid vector, preferably a plasmid vector.

20. The method as claimed in either of claims 18 and 19, **characterized in that** the exogenous enzyme expression vector comprises an antibiotic resistance gene.

21. The method as claimed in claim 20, **characterized in that** the antibiotic resistance gene is a 15 gene for resistance to geneticin (G418), puromycin, blasticidin, hygromycin B, mycophenolic acid or zeocin, preferably a puromycin resistance gene.

**22.** The method as claimed in any one of claims 19 to 21, **characterized in that** the introduction of said expression vector into said cells is carried out by infection with viral particles carrying the exogenous enzyme gene when the expression vector is a viral vector, or by chemical or 20 physical methods when the expression vector is a plasmid vector.

**23.** The method as claimed in claim 22, **characterized in that**, when the vector is a plasmid vector, the introduction into the target cells is carried out by electroporation.

**24.** The use of the method as claimed in any one of the preceding claims, for measuring the antibody-dependent cell-mediated cytotoxicity (ADCC), measuring the complement-dependent 25 cytotoxicity (CDC) and/or measuring apoptosis.

**25.** A kit for carrying out the method as claimed in any one of claims 1 to 16 or the use thereof as claimed in claim 24, **characterized in that** it comprises:

- a line of target cells expressing the exogenous enzyme,
- cytotoxic effector cells for carrying out an ADCC test and/or a source of complement for carrying out a CDC assay,
- a substrate which activates said exogenous enzyme so as to produce an emission of light, and any associated buffer solutions,
- an instruction sheet.

**A**

**B**

FIG. 1    (Art antérieur)

FIG. 2

FIG. 3

FIG. 4

**FIG. 5**

**FIG. 6**

**FIG. 7**

**Raji**

**SKOV3**

FIG. 8

FIG. 9

FIG. 10

FIG. 11

FIG. 12

**FIG. 13**

**FIG. 14**

**FIG. 15**

**FIG. 16**

**FIG. 17**

**FIG. 18**

**FIG. 19**

**FIG. 20**

**FIG. 21**

**FIG. 22**

**FIG. 23**

**FIG. 24**

**FIG. 25**

**FIG. 26**

FIG. 27

**A**

**B**

FIG. 28

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Unconventional sécrétion of the mutated 19kDa protein of Oplophorus luciferase (nanoKAZ) in mammalian cells. **NOUYE SATOSHI et al.** BIOCHEMICAL AND BIOPHYSICAL RESEARCH COMMUNICATIONS. ACADEMIC PRESS INC, 11 Juillet 2014, vol. 450, 1313-1319
- **J. M. COPPOLA et al.** Noninvasive Imaging of Apoptosis and Its Application in Cancer Therapeutics. *CLINICAL CANCER RESEARCH,* 27 Mars 2008, vol. 14, 2492-2501
- **SCHÄFER et al.** *Journal of Immunological Methods,* 1997, vol. 204, 89-98

- **ALPERT et al.** *J. Virol.,* 2012, vol. 86, 12039
- **FU et al.** *PLoS ONE,* 2010, vol. 5, e11867
- **S. INOUYE et al.** Secretional luciferase of the luminous shrimp Oplophorus gracilirostris : cDNA cloning of a novel imidazopyrazinone luciferase. *FEBS Letters,* 2000, vol. 481, 19-25
- **M. HALL et al.** Engineered luciferase reporter from a deep sea shrimp utilizing a novel imidazopyrazinone substrate. *ACS Chemical Biology,* 2012, vol. 7, 1848-1857
- **NORISADA et al.** *Biochem. Biophys. Res. Comm.,* 2014, vol. 449, 483